# EUROPEAN PATENT SPECIFICATION

(11) **EP 2 761 290 B1**
(45) Date of publication and mention of the grant of the patent: **25.10.2017**
(21) Application number: 12759371.3
(22) Date of filing: 12.09.2012
(51) Int. Cl.: G01N 33/50

(54) **METHOD OF DETERMINING THE STATUS OF CELLS.**
VERFAHREN ZUR BESTIMMUNG DES STATUS VON ZELLEN
PROCÉDÉ DE DÉTERMINATION DE L'ÉTAT DE CELLULES.

(30) Priority: 28.09.2011 EP 11007865
(43) Date of publication of application: 06.08.2014
(73) Proprietor: Merck Patent GmbH, 64293 Darmstadt (DE)
(72) Inventor: BECHER, Burkhard, 69221 Dossenheim (DE); HENZLER, Tanja, 68163 Mannheim (DE); HERGET, Thomas, 64285 Darmstadt (DE); WUENSCHE, Verena, 64293 Darmstadt (DE); ZENKE, Frank, 64291 Darmstadt (DE); SCHACHE, Janet, 64319 Pfungstadt (DE)
(86) International application number: PCT/EP2012/003818
(87) International publication number: WO 2013/045033

(56) References cited:
- WO-A1-01/23886
- WO-A2-2007/103374
- O'BRIEN P J ET AL: "High concordance of drug-induced human hepatotoxicity with in vitro cytotoxicity measured in a novel cell-based model using high content screening", ARCHIVES OF TOXICOLOGY, SPRINGER-VERLAG, BERLIN, DE, vol. 80, no. 9, 6 April 2006 (2006-04-06), pages 580-604, XP019419762, ISSN: 1432-0738, DOI: 10.1007/S00204-006-0091-3
- V. C. ABRAHAM ET AL: "Application of a High-Content Multiparameter Cytotoxicity Assay to Prioritize Compounds Based on Toxicity Potential in Humans", JOURNAL OF BIOMOLECULAR SCREENING, vol. 13, no. 6, 1 July 2008 (2008-07-01), pages 527-537, XP055025628, ISSN: 1087-0571, DOI: 10.1177/1087057108318428
- AMACHER ET AL: "The discovery and development of proteomic safety biomarkers for the detection of drug-induced liver toxicity", TOXICOLOGY AND APPLIED PHARMACOLOGY, ACADEMIC PRESS, US, vol. 245, no. 1, 15 May 2010 (2010-05-15), pages 134-142, XP027035321, ISSN: 0041-008X [retrieved on 2010-02-26]
- MICHAEL FRITZSCHE ET AL: "Fluorescent cell-based sensing approaches for toxicity testing", ANALYTICAL AND BIOANALYTICAL CHEMISTRY, SPRINGER, BERLIN, DE, vol. 398, no. 1, 1 April 2010 (2010-04-01) , pages 181-191, XP019839190, ISSN: 1618-2650
- HEWITT PHILIP ET AL: "Value of new biomarkers for safety testing in drug development.", EXPERT REVIEW OF MOLECULAR DIAGNOSTICS SEP 2009 LNKD- PUBMED:19731999, vol. 9, no. 6, September 2009 (2009-09), pages 531-536, XP002683052, ISSN: 1744-8352

## Description

The present invention is directed to a method and assay with which certain cellular parameters can be determined. This method and assay provides an effective means for assessing drug candidate liabilities (toxicities and side effects) to better predict safety early in the drug development process.

The process of drug development is a complex procedure. The average drug takes more than 10 years from initial discovery to public usage. Beginning with preclinical research, new potential drugs are discovered in laboratories and tested in vitro and in animals for safety and biological activity. If a compound is thought to be safe and effective as a drug, clinical studies begin. This is also at least a three-phase process which only very few potential drugs pass until the end. That means those drugs that are approved for marketing comprise an extremely small percentage of all potential drugs that are tested.
In preclinical development, the toxic and pharmacologic effects of the potential drug are evaluated through *in vitro* and *in vivo* laboratory animal testing. Genotoxicity screening is performed, as well as investigations on drug absorption and metabolism, the toxicity of the drug's metabolites, and the speed with which the drug and its metabolites are excreted from the body.
The area of preclinical development is critical to ensuring the safety of all drugs entering clinical trials and ultimately the marketplace. Improvements and innovations in preclinical in vitro assays and animal models would eventually lead to better predictability and thus to lower costs in drug development.
Consequently, there is a high need to provide methods for improving preclinical development so that more information can be gained from the tests and more drug candidates can already be identified as unsuitable for patient treatment.

In vitro cell culture tests are one important means in preclinical development. One can evaluate how a cell interacts with its environment, what functions it performs, what effectors it releases into its environment and what signals it provides to other cells. In order to understand the specific actions and capabilities of a cell, it is desirable to characterize the many factors a cell can produce in a given environment. Typically only a very limited number of cellular functions are tested in preclinical development. To define and establish tests for additional parameters concerning cellular functions and behavior and to provide better means, faster and more efficient prediction of cell behavior, cellular effects and clinical performance is of great interest in a number of fields, including clinical medicine where it can impact upon diagnosis, prognosis and treatment options for disease states such as cancer, autoimmunity, infectious disease and heart disease.
WO 2007/103374 and WO 01/023886 disclose methods for determining a level of toxicity of a chemical compound by culturing cells and measuring at least three indicators of cell health.

In addition there is substantial interest in methods of screening potential new targets and potential drugs for their effectiveness in physiologically relevant situations. Desirable compound screening methods solve this problem by both allowing for a high throughput so that many individual compounds can be tested; and by providing biologically relevant information so that there is a good correlation between the information generated by the screening assay and the pharmaceutical effectiveness of the compound. The development of screening assays that can provide better, faster and more efficient prediction of mechanisms of action, cellular effects and clinical performance is of great interest in drug development, and is addressed in the present invention.

WO 01/57530 discloses a method for functional profiling of cells whereby the cells are arrayed on a substrate through binding to immobilized or partially diffused probes, cells or fragments thereof.

It would be favorable to have an assay that can be performed without immobilizing the cells and that provides a collection of parameters out of which researchers in drug development can select those parameters that are of relevance for testing their specific drug candidates. Many assay formats have been suggested but so far none of these formats provides for a set of parameters that really generates the information necessary to predict drug safety.

It has been discovered that monitoring of a subset of defined cellular parameters represents an effective means to assess unexpected drug candidate toxicities and thereby better predict safety and efficacy early in the drug development process.

Consequently the present invention is directed to a method for determining the status of cells by
a) incubating the cells with the potential drug
b) lysing the cells with a lysis buffer comprising dodecyl-maltoside
c) performing an assay for at least 4 parameters out of the following group of parameters:
   DNA Integrity
   Cell Division
   Cell Death
   Transcriptional Activity
   Protein Biosynthesis
   Mitochondrial Activity
   Stress Response
   Oxidative Stress
   Protein Degradation
   Protein secretion
   Autophagy
   Metabolic activity
   whereby if the parameter DNA integrity is measured it is measured by determining an analyte selected from the group of ATM, ATR, RAD-1, RAD-9, Hus-1, BRCA-1, BRCA-2, ATRIP, RPA-1, KU-70, KU-80, BARD-1, H2A.X (pSer139), CHK1 (pSer296), p53 (pSer15), CHK2 (pThr68), KAP1 (pSer824) and 53BP1 (pThr543),
   if the parameter Cell Division is measured it is measured by determining an analyte selected from the group of Cyclin-D, Cyclin-B, CDC-25A, CDC-25B, Aurora-A, Aurora-B, E2F, Histone H3 (pSer10), Rb (pSer780, pSer795, pSer807/811) and Cdc2 (pTyr15),
   if the parameter Cell death is measured it is measured by determining an analyte selected from the group of Puma, Bax, Bad, Bcl-2, Bcl-XL, Bcl-W, Apaf-1, Smac/Diablo, Noxa, AIF, Mcl-1, Cytochrom c, GADD34, BAG-1, Caspase-7, Caspase-8, Caspase-9, Caspase-10, Histone H2B (pSer14), Caspase-3, cleaved PARP and DFF-45,
   if the parameter Transcriptional Activity is measured it is measured by determining an analyte selected from the group of TFIID, TFIIH, TFIIB, SP1, PTEFb (positive transcription elongation factor b), RNA Pol II (pSer2/5), pan RNAPol II and Mcl-1,
   if the parameter Protein Biosynthesis is measured it is measured by determining an analyte selected from the group of PRKCA, MKNK1, MAPK1, mTOR, DIO2, Akt1, eIF4E, PPP1CA, MAPK3, eIF4G1, HSPB1, PTK2B, MKNK2, eIF2alpha (pSer51), eIF4B (pSer422) and 4E-BP1 (pThr37/46),
   if the parameter Mitochondrial Activity is measured it is measured by determining an analyte selected from the group of enzymes of the citrate cyclus, Complex I, II, III, IV, V, NNT (nicotinamide nucleotide transhydrogenase) and frataxin
   if the parameter Stress Response is measured it is measured by determining an analyte selected from the group of Hsp70, Hsp27, HSP60, HSP90 and Hsp27 (pS78/82),
   if the parameter Oxidative Stress is measured it is measured by determining an analyte selected from the group of PRDX1 (peroxiredoxin 1), GPX1, ANXA1, CCND1, DERL2, ERN1, ERO1L, GPX1, GSK3B, HDAC1, HERPUD1, HMOX1, HSPA5, LONP1, PPP1R15A, PRDX2, PXN, SPHK1, TXNRD1, WFS1, HIF-1alpha and Ho-1,
   if the parameter Protein Degradation is measured it is measured by determining Polyubiquitin (Lys48),
   if the parameter Protein secretion is measured it is measured by determining an analyte selected from the group of protein disulfide isomerase (PDI), Hsc70, calnexin, calreticulin, peptidyl-prolyl isomerase, N-acetylgalactosamine (GaINAc) transfer and CHOP,
   if the parameter Autophagy is measured it is measured by determining an analyte selected from the group of Beclin-1, VPS-34, PTEN, mTOR, PI-3 kinase, ULK-1, ULK-2, FIP-200, ATG-101, APG-3, APG-7, APG-12, LC3 and NBR-1,
   if the parameter Metabolic Activity is measured it is measured by determining an analyte selected from the group of AMPK (pThr172), Acetyl-CoA carboxylase (pSer79) and PDHE1 (pSer232, pSer239 pSer300) and whereby a multiplexed assay format is used.

In a preferred embodiment, at least 6 parameters are selected out of the group of parameters given above.

In a preferred embodiment, the parameter DNA Integrity is measured by determining the level of phosphorylation on serine 139 of γH2A.X as an analyte for DNA Integrity.

In a preferred embodiment, the parameter Cell Cycle status is measured by determining the Histone H3 (pSer10) content as an analyte for Cell Cycle. In a preferred embodiment, the parameter Cell Death status is measured by determining cleaved PARP.

In a preferred embodiment, the parameter Transcriptional Activity is measured by determining the amount of DNA-dependent RNA Polymerase II (RNA Pol II) that is phosphorylated at CTD serine 2/5.

In a preferred embodiment, the parameter Protein Biosynthesis is measured by determining the amount of eIF4B (pSer422).

In a preferred embodiment, the parameter Mitochondrial Activity biosynthesis is measured by determining the Complex IV level.

In a preferred embodiment, the parameter Stress Response biosynthesis is measured by determining the HSP70 level.

In a preferred embodiment, the parameter Oxidative Stress biosynthesis is measured by determining the expression of HIF-1α.

In a preferred embodiment, the parameter Protein Degradation is measured by determining the level of Polyubiquitin (Lys48).

In a preferred embodiment, the parameter Protein Secretion is measured by determining the expression of CHOP.

In a preferred embodiment, the parameter Autophagy is measured by determining the conversion of LC3-I to LC3-II or total LC3.

In a preferred embodiment, the assay is a bead-based assay.

In a very preferred embodiment, the assay is a bead-based assay for at least 4 parameters out of the following group
DNA Integrity
Cell Division
Cell Death
Transcriptional Activity
Protein Biosynthesis
Mitochondrial Activity
Stress Response
Oxidative Stress
Protein Degradation
Protein secretion
Autophagy
Metabolic activity
whereby
if the parameter DNA Integrity is measured it is measured by determining the level of phosphorylation on serine 139 of γH2A.X as an analyte for DNA Integrity,
if the parameter Cell Cycle status is measured it is measured by determining the Histone H3 (pSer10) content as an analyte for Cell Cycle, if the parameter Cell Death status is measured it is measured by determining cleaved PARP,
if the parameter Transcriptional Activity is measured it is measured by determining the amount of DNA-dependent RNA Polymerase II (RNA Pol II) that is phosphorylated at CTD serine 2/5,
if the parameter Protein Biosynthesis is measured it is measured by determining the amount of eIF4B (pSer422),
if the parameter Mitochondrial Activity biosynthesis is measured it is measured by determining the Complex IV level,
if the parameter Stress Response biosynthesis is measured it is measured by determining the HSP70 level,
if the parameter Oxidative Stress biosynthesis is measured it is measured by determining the expression of HIF-1α,
if the parameter Protein Degradation is measured it is measured by determining the level of Polyubiquitin (Lys48),
if the parameter Protein Secretion is measured it is measured by determining the expression of CHOP,
if the parameter Autophagy is measured it is measured by determining the conversion of LC3-I to LC3-II or total LC3.

In preferred embodiment, an assay format according to the Luminex technology is used.

In a preferred embodiment, in step c) the assays are performed with one or more concentrations of the potential drug and/or for one or more incubation times and/or with at least two or more potential drugs and/or with one or more different cell lines and/or in comparison to a vehicle control.

In a preferred embodiment, at least a second set of data for at least one compound with known effect is generated using the same assay and the results of both assays are compared.

In a preferred embodiment, in a step d) the set of data generated with the assay in step c) is compared with another set of data generated with the assay in step c) or is compared with the sets of data contained in a data base.

In another preferred embodiment in step b) the cells are lysed with a lysis buffer comprising a protease, a phosphatase inhibitor and a nuclease.

In another preferred embodiment in step b) the cells are lysed with a lysis buffer comprising
- 1 to 100 mM dodecyl-maltoside
- 20 to 600 mM magnesium chloride or a mixture of magnesium chloride with chlorides of one or more other alkali or alkaline earth metals, preferably a mixture of 0.5 to 5 mM magnesium chloride and 200 to 500 mM sodium chloride
- at least 0.025 units of a nuclease
- a protease
- a phophatase inhibitor

Also disclosed is an assay format comprising means for determining at least 4 parameters out of the following group of parameters:
DNA Integrity
Cell Division
Cell Death
Transcriptional Activity
Protein Biosynthesis
Mitochondrial Activity
Stress Response
Oxidative Stress
Protein Degradation
Protein Secretion
Autophagy
Metabolic Activity

In a preferred embodiment, the assay is in a multiplexed format.

In a preferred embodiment the assay format comprises at least
- an analyte capture bead mix or single analyte capture beads
- a detection antibody mix or single detection antibodies
- one or more of the following reagents: lysis buffer, phosphatase inhibitors, protease inhibitors, benzonase

The present invention is also directed to the use of the method according to the invention to assess toxicity and/or side effects of potential drugs.

Before describing the present invention in detail, it is to be understood that this invention is not limited to specific compositions or process steps, as such may vary. It must be noted that, as used in this specification and the appended claims, the singular form "a", "an" and "the" include plural referents unless the context clearly dictates otherwise. Thus, for example, reference to "a ligand" includes a plurality of ligands and reference to "an antibody" includes a plurality of antibodies and the like.

Unless defined otherwise, all technical and scientific terms used herein have the same meaning as commonly understood by one of ordinary skill in the art to which this invention is related. The following terms are defined for purposes of the invention as described herein.

An assay is an estimation method or test for the analysis of a certain substance. In the pharmaceutical industry a standardized application flow of a special method for the detection of a certain substance within a sample is termed assay. Bioassays and immunoassays are among the many varieties of specialized biochemical assays. Other assays measure processes such as enzyme activity, antigen capture, stem cell activity, and competitive protein binding.

A **bead based assay** is an assay to detect or measure the presence of an analyte in a sample, whereby the sample is incubated with at least one type of beads carrying capture groups and/or a label.

As used herein, and unless stated otherwise, the term **"**capture **group**" designates an agent able to interact specifically with an "analyte" that is part of the sample or with an interfering substance that is able to specifically interact with an analyte. Any reagent that possesses a high degree of specificity and affinity for the analyte or the interfering substance can be used as capture group. A capture group according to the present invention may be a member of a binding pair, i.e. two molecules, usually two different molecules, that through chemical or physical means specifically bind to each other. The complementary members of a specific binding pair are sometimes referred to as a ligand and receptor; or receptor and counter-receptor. Specific binding pairs and thus pairs of analyte/capture group include carbohydrates and lectins; complementary nucleotide sequences (e.g. DNAs, RNAs, oligonucleotides or analogs thereof, PCR products, genomic DNA, bacterial artificial chromosomes, plasmids and the likes); peptide ligands and receptors; effector and receptor molecules; hormones and hormone binding protein; enzyme cofactors and enzymes; enzyme inhibitors and enzymes; peptides, lipoproteins and their receptors; antibodies, antibody fragments, immunoglobulins, and peptide/MHC complexes. It also includes molecular compounds such as metabolites as well as cellular organelles, intact cells, and the likes.
The specific binding pairs may include analogs, derivatives and fragments of the original specific binding member. For example, a receptor and ligand pair may include peptide fragments, chemically synthesized peptidomimetics, labeled protein, derivatized protein, etc.

As used herein, the term "**label**" refers to a composition detectable by spectroscopic, fluorimetric, photochemical, biochemical, immunochemical, enzymatic, chemical, or other physical means. For example, useful labels include such as but not limited to luminescent molecules (e.g. fluorescent agents , phosphorescent agents, chemiluminescent agents, bioluminescent agents and the likes), coloured molecules, molecules producing colours upon reaction, enzymes, magnetic beads, radioisotopes, specifically bondable ligands, microbubbles detectable by sonic resonance, biotin, digoxigenin, or haptens and proteins or other entities which can be made detectable, e.g., by incorporating a radiolabel into a peptide or used to detect antibodies specifically reactive with an antigen, and the likes.

As used herein, and unless stated otherwise, the term "**analyte**" designates a substance, compound, or composition fixed as goal or point of analysis, that means whose presence, absence, amount, or concentration in a sample or specimen is to be detected or determined. It includes molecular compounds such as but not limited to nucleic acids and related compounds (e.g. DNAs, RNAs, oligonucleotides or analogs thereof, PCR products, genomic DNA, bacterial artificial chromosomes, plasmids and the likes), proteins and related compounds (e.g. polypeptides, monoclonal antibodies, receptors, transcription factors, and the likes), antigens, ligands, haptens, carbohydrates and related compounds (e.g. polysacharides, oligosacharides and the likes), metabolites, cellular organelles, intact cells, and the likes.

As used herein, and unless stated otherwise, the term "sample" refers to any composition or mixture that may contain a target "analyte". Samples may be derived from biological or other sources. Biological sources include eukaryotic and prokaryotic sources, such as plant and animal cells, tissues and organs. The sample may also include diluents, buffers, detergents, and contaminating species, debris and the like that are found mixed with the target analyte.

According to the present invention, a "**cell**" may be a human cell, a mammalian cell (e.g. mouse, monkey, hamster, rat, rabbit or other species), a plant protoplast, yeast, fungus, or any other cell type of interest. The cell can also be a cell line or a primary cell. The cells may also be genetically altered, e.g. by transfection or transduction with recombinant genes or by antisense technology. The cells may be obtained from any mammalian source that is amenable to primary culture and/or adaptation into cell lines. In lieu of generating cell lines from animals, such cell lines may be obtained from, for example, American Type Culture Collection, (ATCC1 Rockville, Md.), or any other Budapest treaty or other biological depository. The cells used in the assays may be from an animal source or may be recombinant cells tailored to express a particular characteristic of, for example, a particular disorder for which the drug development is being considered. In one embodiment, the cells are derived from tissue obtained from humans or other primates, rats, mice, rabbits, sheep, dogs and the like. Techniques employed in mammalian primary cell culture and cell line cultures are well known to those of skill in that art. In the case of commercially available cell lines, such cell lines are generally sold accompanied by specific directions of growth, media and conditions that are preferred for that given cell line. The cell can also be a component of an intact tissue or animal, or in the whole body; or can be isolated from a biological sample or organ. The present invention could also be used in fungal cells to identify antifungal agents that block key pathways or in bacterial cells to identify and characterize antibiotics.

Examples of suitable cells and cell lines are Hek293, HepG2, HeLa, HepaRG, primary liver and kidney cells and liver and kidney nerve cells. Preferred is the use of cells or cell lines which show metabolic activity, e.g. HepaRG. This offers the possibility to also measure the toxicity of metabolites.

A potential drug is any known drug or drug candidate or any substance that is tested for its effect when used as a drug.
Typically a potential drug is a substance or a mixture of substances that have an effect on the function of the body and are used in the treatment, cure, prevention, or diagnosis of disease or used to otherwise to enhance physical or mental well-being.

Figures 1 to 17 show experimental results referred to in the Examples.

Figures 18 and 19 show possible read-outs and data base comparisons of the assay results of a potential drug that are compared with the assay results of other potential drugs and/or drugs with known effect that have also been assayed and whose assay results are stored in a data base.

Drugs, when administered to patients, enter the circulation and if they have adequate pharmacokinetic properties reach various organs and tissues and cells of the body. Consequently, drugs act upon cells, which are the smallest living components of the human body. Typically, drugs are administered to a patient because they have a certain effect on the cells and thus on the patient. Most drugs do not only have one effect, but also cause a plurality of side-effects. A side effect is any activity of a drug or compound on a cellular target or pathway other than the intended effect of the compound. These side-effects can be desirable or undesirable. Undesirable side-effects are those that contribute to the toxicity or long-term adverse effects of a drug.
Ideally, side-effects of a potential drug should be evaluated in the early phases of drug discovery to avoid failures at later, more costly, stages of drug development and, more importantly, to reduce the probability of fatal toxicities in clinical trials. Since the knowledge on the mechanism of action of early drug candidates and especially its safety profile is rudimentary it would be of great help to generate a tool which is capable to assess the impact of novel substances on a comprehensive, meaningful set of cellular processes, which represent housekeeping functions of an intact, healthy cell.

It has now been found that a combination of parameters like DNA Integrity, Cell Division, Cell Death, Transcriptional Activity, Protein Biosynthesis, Mitochondrial Activity, Stress Response, Oxidative Stress, Protein Degradation, Protein Secretion, Metabolic Activity or Autophagy, in the following called the inventive parameters, that are measured according to the method of the present invention provide an effective and reliable set of parameters to determine the side effects of potential drugs. It is also possible to only measure 4, 5, 6, 7, 8, 9, 10 or 11 of these parameters instead of determining all of them.

The present invention is directed to a method for determining the status of cells by
a) incubating the cells with the potential drug
b) optionally lysing the cells
c) performing an assay for at least 4 parameters out of the following group of parameters:
   DNA Integrity
   Cell Division
   Cell Death
   Transcriptional Activity
   Protein Biosynthesis
   Mitochondrial Activity
   Stress Response
   Oxidative Stress
   Protein Degradation
   Protein Secretion
   Autophagy
   Metabolic Activity

In the method according to the present invention, the status of the cell, that means the effect of altering the environment of the cells e.g. by adding a potential drug is assessed by detecting and/or monitoring certain output parameters. Indicators for these parameters are typically analytes which change their expression level or posttranslational modifications such as phosphorylation or glycosylation in case the parameter is affected. The results with different cells and/or different parameters and/or different compounds can be compared as to their similarities and differences.

A potential drug is any chemical or biological compound or other stimulus that is brought in contact with the cells and whose effect on the cells is detected by performing an assay for certain inventive parameters. A potential drug can be a single compound or a mixture of two or more compounds.

By performing an assay for certain inventive parameters and thus generating a set of data representative for the effect of a certain potential drug on the cells, one can generate a data set that is unique for each potential drug with respect to a given cell type or family of cells and certain assay conditions. Comparison of the profiles of a potential drug with a set of known pharmacologically-important compounds and/or with other potential drug and/or compounds with known effects on cells can provide for an efficient and expeditious approach to identify drug candidate toxicity to better predict safety early in the drug development process.

The method according to the invention and thus performing an assay for at least 4 inventive parameters can be done with any method known in the art.

Suitable assay formats are for example:
- immuno assays, e.g. sandwich immuno assays
- assays based on protein-protein interactions
- assays based on enzyme reactions like kinase-assays
- assays based on interactions between nucleic acids, like DNA-DNA interactions, DNA-RNA-interactions, RNA-RNA-interactions
- assays based on DNA-protein interactions
- assays based on peptide-protein or peptide-peptide interactions
- assays based on glycan-protein interactions
- assays based on metabolite-protein interactions
- assays based on metabolite-DNA interactions
- assays based on metabolite-RNA interactions

All of these assays are typically based on the detection and/or measurement of a certain analyte. The analyte can be an individual cellular component, such as the presence or absence of an antigen, alternatively, it can be based upon a biological response, such as a change in second messenger, or electrical activity. Examples of analytes are DNA, RNA, and proteins. In the method according to the present invention a certain set of inventive parameters is determined by performing a certain set of assays providing information about the presence and/or concentration of specific analytes.
This is done during a single experiment by using so-called multiplexed microarray or multiplexed bead-based techniques.

Various methods can be utilized for detecting and/or quantifying the presence of an analyte. For measuring the amount of a molecule that is present, a convenient method is to label a molecule with a detectable moiety, which may be fluorescent, luminescent, radioactive, enzymatically active, etc., particularly a molecule specific for binding to the analyte with high affinity. Fluorescent moieties are readily available for labeling virtually any biomolecule, structure, or cell type. Immunofluorescent moieties can be directed to bind not only to specific proteins but also specific conformations, cleavage products, or site modifications like phosphorylation. Individual peptides and proteins can be engineered to autofluorescence, e.g. by expressing them as green fluorescent protein chimeras inside cells (for a review see Jones et al. (1999) Trends Biotechnol. 17(12):477-81). Thus, antibodies can be genetically modified to provide a fluorescent dye as part of their structure.

Other fluorescent sensors have been designed to report on biological activities or environmental changes, e.g. pH, calcium concentration, electrical potential, proximity to other probes, etc. Methods of interest include calcium flux, nucleotide incorporation, quantitative PAGE (proteomics), etc.

Highly luminescent semiconductor quantum dots (zinc sulfide-capped cadmium selenide) have been covalently coupled to biomolecules for use in ultrasensitive biological detection (Stupp et al. (1997) Science 277(5330):1242-8; Chan et al. (1998) Science 281(5385):2016-8). Compared with conventional fluorophores, quantum dot nanocrystals have a narrow, tunable, symmetric emission spectrum and are photochemically stable (Bonadeo et al. (1998) Science 282(5393):1473-6). The advantage of quantum dots is the potential for exponentially large numbers of independent readouts from a single source or sample.

Multiple fluorescent labels can be used on the same sample and individually detected quantitatively, permitting measurement of multiple cellular responses simultaneously. Many quantitative techniques have been developed to harness the unique properties of fluorescence including: direct fluorescence measurements, fluorescence resonance energy transfer (FRET), fluorescence polarization or anisotropy (FP), time resolved fluorescence (TRF), fluorescence lifetime measurements (FLM), fluorescence correlation spectroscopy (FCS), and fluorescence photobleaching recovery (FPR) (Handbook of Fluorescent Probes and Research Chemicals, Seventh Edition, Molecular Probes, Eugene Oreg.).

Depending upon the label chosen, parameters may be measured using other than fluorescent labels, using such immunoassay techniques as radioimmunoassay (RIA) or enzyme linked immunosorbance assay (ELISA), homogeneous enzyme immunoassays, and related non-enzymatic techniques. These techniques utilize specific antibodies as reporter molecules, which are particularly useful due to their high degree of specificity for attaching to a single molecular target. U.S. Pat. No. 4,568,649 describes ligand detection systems, which employ scintillation counting. These techniques are particularly useful for protein or modified protein parameters or epitopes, or carbohydrate determinants. Cell readouts for proteins and other cell determinants can be obtained using fluorescent or otherwise tagged reporter molecules. Cell based ELISA or related non-enzymatic or fluorescence-based methods enable measurement of cell surface parameters and secreted parameters. Capture ELISA and related non-enzymatic methods usually employ two specific antibodies or reporter molecules and are useful for measuring parameters in solution. Flow cytometry methods are useful for measuring cell surface and intracellular parameters, as well as shape change and granularity and for analyses of beads used as antibody- or probe-linked reagents. Readouts from such assays may be the mean fluorescence associated with individual fluorescent antibody-detected cell surface molecules or cytokines, or the average fluorescence intensity, the median fluorescence intensity, the variance in fluorescence intensity, or some relationship among these.

The method according to the invention is performed in a multiplexed assay format. That means two or more inventive parameters can be simultaneously determined in one sample in a single assay.

In a multiplexed format, the readouts of selected parameters are capable of being read simultaneously, or in sequence during a single analysis, as for example through the use of fluorescent antibodies to cell surface molecules. As an example, these can be tagged with different fluorochromes, fluorescent beads, tags, e.g. quantum dots, etc., allowing analysis of up to 4 or more fluorescent colours simultaneously by various flow cytometry techniques.
Nucleic acids, e.g. messenger RNAs, can be measured by hybridization techniques that depend on the sequence of the nucleic acid nucleotides. Techniques include polymerase chain reaction methods as well as gene array techniques.

One technique for performing multiplexed immunoassays is the Luminex xMAP® technology which is based on beads which are colour coded with at least two different fluorescent dyes. The beads are typically plastic beads (e.g. polystyrene beads) or magnetic beads. Preferred are magnetic beads. The combination of different concentrations of these at least two fluorescent dyes ends up in more than 100 different bead types. A third fluorescent dye can be used as reporter. The reporter fluorescent is an indicator for a positive reaction on the bead surface.

For performing a sandwich immuno assay in a multiplex fashion using the Luminex xMAP® technology the capture antibody specific for an analyte is coupled to one type of fluorescent-labeled beads. The analyte captured by the bead-coupled antibody can be detected by a second antibody whereby the second antibody directly or indirectly produces a detection signal. In consequence, with this type of multiplexed assay format several conventional ELISA assays can be done in one well in parallel.

As an example, Luminex® beads or other fluorescent beads, or beads varying in light scattering parameters can be conjugated to antibodies to cytokines or other analytes, or conjugated to protein receptors for analytes. The conjugated beads are added to the cells, cell lysate, or to the removed supernatant, allowing bead binding to target analytes. Also, a fluorescent antibody to a distinct epitope of the target analyte is used to measure the level of target analyte bound. The fluorescence and light scatter characteristics of the beads constitute an identifier of the target analyte, and fluorescence derived from added antibody to the target analyte is an indication of the quantity of analyte bound, and hence a readout of the individual analyte.

Flow cytometry may be used to quantitate parameters such as the presence of cell surface proteins or conformational or posttranslational modification thereof; intracellular or secreted protein, where permeabilization allows antibody (or probe) access, and the like.

### Assay performance

To perform the method of the present invention, one has to provide the cells that shall be incubated with the potential drug and the potential drug itself. The potential drug is typically applied in an aqueous solution. If the potential drug is not water-soluble it might be dissolved in an organic solvent like DMSO, ethanol or methanol or mixtures thereof or in a mixture of water or aqueous buffer with one or more water-miscible organic solvents which do not negatively influence the cell culture and/or the analysis of the parameters.
A wide variety of cells may be utilized with the present invention. The choice of cells selected will depend on the particular potential drug and the application of interest. One skilled in the art knows that each type of cell may require a particular isolation procedure and culture condition for growth and proliferation.
The cells may e.g. be maintained in culture prior to performing the method according to the invention, may be isolated from human blood or tissues according to standard methods or may be recovered from low temperature storage in an appropriate media.

For performing the method according to the present invention, the cells are then incubated with the potential drug. When using cell cultures, the cells are typically cultured under conditions suitable for the chosen cell line. A person skilled in the art is able to choose suitable conditions. Often it is favourable to culture the cells until sub-confluence. Typically the incubation is performed for different times and with different amounts of the potential drug. Preferably, these incubations are performed in parallel e.g. in multiwell plates or equivalent equipment. The cells are then incubated with the potential drug for a certain time. The duration of the incubation may depend on the potential drug to be tested, the cells and the parameters to be determined. Typical incubation times are between 30 minutes and 7 days.
Typically the incubation is done under defined conditions like temperature, CO₂ etc. that allow for optimal growth of the cells. Typical culture conditions for human cell culture are 5-10% CO₂, 89% humidity, 37°C. For certain incubations it might be advisable to induce stress to the cells by altering the culture conditions. A person skilled in the art knows suitable ways of modifying the culturing and incubation conditions. Typically also a vehicle control without compound is performed.

After the incubation, depending on the parameter and the respective analyte to be determined, the cells and/or the cell culture supernatant are further analyzed. For the determination of most of the parameters, the cells are lysed prior to further analysis. Methods for lysing cells are known to a person skilled in the art, examples are thermal lysis, mechanical lysis or preferably chemical lysis by adding a suitable lysis buffer. Suitable lysis buffers to be used according to the method of the present invention are buffers comprising NP40, TritonX100, Tween20, sodium deoxycholate or dodecyl-maltoside, IGEPAL, SDS. The lysis buffers comprise dodecyl-maltoside. Also preferred are lysis buffers in which chlorides of one or more alkali or alkaline earth metals, especially sodium chloride, are present in concentrations between above 200 mM, preferably between 200 and 600 mM. Also preferred are lysis buffers which comprise dodecyl-maltoside and chlorides of one or more alkali or alkaline earth metals, especially sodium chloride, whereby the chlorides are present in concentrations above 200 mM, preferably between 200 and 600 mM. Suitable exemplary compositions of the buffers can be found in the examples. Preferably, the lysis buffer also comprises one or more of the following supplements: protease inhibitors (e.g. Protease inhibitor cocktail (EMD Chemicals)), phosphatase inhibitors (e.g. Phosphatase Inhibitor. Cocktail V (EMD Chemicals)), a nuclease (preferably Benzonase ® (EMD Chemicals)), magnesium chloride and sodium chloride. In a very preferred embodiment, the lysis buffer comprises a protease inhibitor, a phosphatase inhibitor, dodecyl-maltoside, a nuclease and magnesium chloride or a mixture of magnesium chloride with chlorides of one or more other alkali or alkaline earth metals.
In this preferred lysis buffer, dodecyl-maltoside is typically present in a concentration between 1 and 100 mM, preferably between 5 and 50 mM, very preferred it is present in a concentration between 25 and 35 mM. Magnesium chloride or a mixture of magnesium chloride with chlorides of one or more other alkali or alkaline earth metals is typically present in concentrations between 20 and 600 mM, preferably between 200 and 500 mM, most preferred between 300 and 400 mM. Preferred is a combination of magnesium chloride and one or more other alkali or alkaline earth metals like sodium chloride, potassium chloride or calcium chloride. In a very preferred embodiment, magnesium chloride is present in concentrations between 0.5 and 5 mM, preferably between 1 and 2 mM, and sodium chloride is present in concentrations between 20 and 600 mM, preferably between 200 and 500 mM, most preferred between 300 and 400 mM.
The nuclease is typically present in at least 0.025 units, preferably in 0.1 to 0.5 units, most preferred in 0.2 to 0.3 units. The nuclease preferably is an endonuclease, very preferred it is an endonuclease derived from a *Serratia marcescens* endonuclease like Benzonase(R).
The pH of the lysis buffer is typically between pH 6.9 and 8, preferably between 7 and 7.6.

Consequently, the most preferred lysis buffer comprises dodecyl-maltoside in a concentration between 25 and 35 mM, magnesium chloride in a concentration between 1 and 2 mM, sodium chloride in a concentration between 300 and 400 mM, 0.2 to 0.3 units of an endonuclease, a protease inhibitor and a phosphatase inhibitor and has a pH between 7 and 7.6. After lysis, the cell lysates are typically cleared by filtration or by centrifugation. The supernatant is then used for further analysis.

The selected parameters and respective analytes can then be assayed by any known method. Typically the analytes are captured and detected with the respective antibodies as capture groups.
Preferred capture groups include antibodies and fragments thereof, which bind to a certain analyte or set of analytes. The affinity and specificity of the binding members must be sufficient to assay changes in the concentration of the analyte in the sample using a suitable assay method.

Preferred methods are Western Blot analyses, ELISA or other immunoassays. In a very preferred embodiment, the assay is performed with a multiplexed immunoassay so that two or more or even all parameters can be determined simultaneously in one assay. In an especially preferred embodiment, the parameters are assayed by a bead-based immuno assays e.g. using the Luminex xMAP® technology. In bead based immuno assays a capture antibody specific for an analyte is typically coupled to one type of fluorescent-labeled beads. The analyte captured by the bead-coupled antibody can be detected by a second antibody whereby the second antibody directly or indirectly produces a detection signal.
The methods of performing a multiplexed assay are known to a person skilled in the art. Typically, the cell lysate is incubated with the beads carrying the capture groups. After washing the beads, they are mixed with detection antibodies and after incubation washed again. Afterwards, depending on the type of the beads a detection signal is measured. In one embodiment, the detection antibodies which are biotinylated are incubated with streptavidine - phycoerythrine and are then measured for detection. Other methods than Luminex suitable for the detection of the analytes are:
- multiplex ELISAs (planar protein arrays)
- Mass Spectrometry
- NMR
- methods to detect specific proteins or nucleic acid at the single molecule level using assembled nanodevices that include transcriptional regulator proteins and a F1-ATPase molecular motor. Single molecules of this molecular motor can be detected by dark field microscopy when the F1-ATPase is attached to the surface of a microscope slide via his-tags on the beta subunits. This attachment geometry positions the end of the gamma subunit that protrudes from the ring of alpha and beta subunits away from the surface to allow attachment to a single gold nanorod by an Avidin-biotin linkage. Addition of ATP provides fuel to the motor such that ATP hydrolysis powers the rotation of the attached nanorod. This rotation is easily observed as a blinking of red and greed scattered light from the nanorod. Multiplex detection of several analytes is possible with this technique. Binding of analyte to binding protein results in the induction of a conformational change of the binding protein. This conformational change generates a new binding site for a probe e.g. labeled nucleic acid complex or protein which can be measured.
- multiplex or parallel singleplex detection by using an electrochemical device

### Parameters

### DNA integrity:

Eukaryotic cells have developed a network of highly conserved surveillance mechanisms called checkpoints (see also cell division) which ensure that damaged chromosomes are repaired before they are replicated or segregated during the cell cycle. The tumor suppressor protein p53 is regarded as a 'master tumor suppressor' which ensures the integrity of the cells' genome by protecting it from adverse effects of DNA damage. The underlying mechanisms are essential for maintaining genomic integrity and cell viability. DNA damage will be repaired during a growth arrest, or the damaged cells will be eliminated, thereby preventing fixation of DNA damage as mutations [Albrechtsen N, Dornreiter I, Grosse F, Kim E, Wiesmüller L Wolfgang Deppert W (1999). Maintenance of genomic integrity by p53: complementary roles for activated and non-activated p53. Oncogene. (18): 7706 - 7717].

Indicators for this parameter are analytes which change their expression level or posttranslational modifications such as phosphorylation or glycosylation in case a compound has an effect on the parameter.

Genotoxic agents might induce DNA damage. The response of eukaryotic cells to double-strand breaks in genomic DNA includes the sequestration of many factors into nuclear foci. Certain proteins become extensively phosphorylated upon DNA damage. These proteins are suitable analytes for determining the parameter "DNA integrity" as anitbodies directed against these proteins can be used to detect and measure these proteins. Other analytes for DNA integrity are proteins involved in the DNA repair pathway, like BRCA-1, BRCA-2, KU-70 and KU-80 or proteins involved in the cell cycle arrest in response to DNA damage like Hus-1. Preferred analytes are those that are activated, e.g. by phosphorylation, in case of DNA damage. Examples of possible analytes for DNA integrity are ATM, ATR, RAD-1, RAD-9, Hus-1, BRCA-1, BRCA-2, ATRIP, RPA-1, KU-70, KU-80, BARD-1, preferably H2A.X (pSer139), CHK1 (pSer296), p53 (pSer15), CHK2 (pThr68), KAP1 (pSer824), 53BP1 (pThr543). Especially preferred is H2A.X (pSer139).
Histone H2A.X is a 14 kDa ubiquitous member of the H2A histone family. It contains an evolutionary conserved SQ motif at the C-terminus in eukaryotes. Serine 139 within this motif becomes rapidly phosphorylated to yield a form known as γ-H2A.X (gamma-H2A.X) in response to double-strand DNA damage. H2A.X becomes extensively phosphorylated within 1-3 minutes of DNA damage and forms foci at break sites.

### Cell Division:

Cell division is part of the life cycle of a cell. A new cell arises when one cell divides into two cells and requires the start of a cell-replication program. This program involves a period of cell growth, during which proteins are made and DNA is replicated, followed by cell division, where the cell divides into daughter cells. Most eukaryotic cells proceed through a sequence of phases, called the cell cycle. During the cell cycle DNA is duplicated in the synthesis phase and the copies are partitioned during the mitotic phase. The cell cycle is controlled by checkpoints which monitor the fidelity of the whole process [Vermeulen K, Van Bockstaele DR and Berneman ZN. (2003). The cell cycle: a review of regulation, deregulation and herapeutic targets in cancer. Cell Prolif. 36, 131-1494,5 and Lodish H. Berk, A. Zipursky S.L., Matsudeira P, Baltimore D., Darnell J. (2000). Molecular Cell Biology. fourth edition, W.H. Freeman and Company*].*

Indicators for this parameter are analytes which change their expression level or posttranslational modifications such as phosphorylation or glycosylation in case the parameter is affected .

The mechanism of cell cycle and cell division is known to the person skilled in the art. Two key classes of regulatory molecules, cyclins and cyclin-dependent kinases (CDKs), determine a cell's progress through the cell cycle. Cyclins form the regulatory subunits and CDKs the catalytic subunits of an activated heterodimer; cyclins have no catalytic activity and CDKs are inactive in the absence of a partner cyclin. CDKs are constitutively expressed in cells whereas cyclins are synthesised at specific stages of the cell cycle. Other suitable analytes for cell division are proteins involved in mitosis. They can also be used as marker for the mitotic fraction of the cells. For example, cell cycle arrest is indicated by reduction or increase on Histone H3 (pSer10) content.
Examples for suitable analytes for cell division are Cyclin-D, Cyclin-B, CDC-25A, CDC-25B, Aurora-A, Aurora-B, E2F-, preferably Histone H3 (pSer10), Rb (pSer780, pSer795, pSer807/811), Cdc2 (pTyr15), very preferred Histone H3 (pSer10).

### Metabolic activity:

Metabolism is the plethora of chemical reactions in living organisms to maintain life. Catabolism breaks down molecules into smaller units and provides energy, e.g. in the form of adenosine triphosphate ATP [Campbell NA, Reece JB. Biologie. (2002) 6. Edition, Spektrum Akademischer Verlag]. In catabolism, large molecules (polymers) such as polysaccharides, lipids, nucleic acids and proteins are broken down into smaller units and monomers such as monosaccharides, fatty acids, nucleotides, and amino acids, respectively. Cells use the monomers to construct new polymer molecules, or degrade the monomers further to simple waste products, releasing energy. Cellular wastes include lactic acid, acetic acid, carbon dioxide, ammonia, and urea. Anabolism is the set of metabolic pathways that construct molecules (polysaccharides, lipids, nucleic acids and proteins) from smaller units. These reactions require energy. The energy status is an important indicator of the metabolic status and activity of a cell.

Indicators for this parameter are analytes which change their expression level or posttranslational modifications such as phosphorylation or glycosylation in case the parameter is affected.
Examples for suitable analytes for metabolic activity are AMPK (pThr172), Acetyl-CoA carboxylase (pSer79), PDHE1 (pSer232, pSer239 and pSer300), preferred is Acetyl-CoA carboxylase (pSer79).

### Cell Death:

Apoptosis is a genetically encoded programme leading to cell death that is involved in normal development and homeostasis of multicellular organisms. [Tait SW, Green DR. Mitochondria and cell death: outer membrane permeabilization and beyond. Nat Rev Mol Cell Biol. 2010 Sep;11(9):621-32. Epub 2010 Aug 4. Review] Apoptosis is an irreversible process and once initiated leads to characteristic cell changes and death. These changes include blebbing, loss of cell membrane asymmetry and attachment, cell shrinkage, nuclear fragmentation, chromatin condensation, and chromosomal DNA fragmentation [Alberts, Keith; Johnson, Alexander; Lewis, Julian; Raff, Martin; Roberts; Walter, Peter (2008). "Chapter 18 Apoptosis: Programmed Cell Death Eliminates Unwanted Cells". Molecular Biology of the Cell (textbook) (5th ed.). Garland Science. p. 1115. ISBN 978-0-8153-4105-5]. The process of apoptosis is controlled by a diverse range of cell signals which may originate either extra or intracellularly.
Indicators for this parameter are analytes which change their expression level or posttranslational modifications such as phosphorylation or glycosylation in case the parameter is affected.

Examples for suitable analytes for cell death are Puma, Bax, Bad, Bcl-2, Bcl-XL, Bcl-W, Apaf-1, Smac/Diablo, Noxa, AIF, Mcl-1, Cytochrom c, GADD34, BAG-1, Caspase-7, Caspase-8, Caspase-9, Caspase-10, preferably Histone H2B (pSer14), Caspase-3, cleaved PARP, DFF-45, most preferred is cleaved PARP.

### Autophagy:

Autophagy is an evolutionary conserved, catabolic process that involves the entrapment of cytoplasmic components within characteristic vesicles for their delivery to and degradation within lysosomes. Depending on the route of delivery to the lysosome three different types of autophagy are defined: microautophagy, chaperone-mediated autophagy and macroautophagy. The role of autophagy extends beyond the general homeostatic removal, degradation and recycling of damaged proteins and organelles to many specific physiological and pathological processes such as development, immunity, energy homeostasis, cell death, tumourigenesis and many more [Rosenfeldt MT, Ryan KM. (2011). The multiple roles of autophagy in cancer. Carcinogenesis*.* [Epub ahead of print]].

Indicators for this parameter are analytes which change their expression level or posttranslational modifications such as phosphorylation or glycosylation in case the parameter is affected.

Examples for suitable analytes for autophagy are Beclin-1, VPS-34, PTEN, mTOR, PI-3 kinase, ULK-1, ULK-2, FIP-200, ATG-101, APG-3, APG-7, APG-12, preferably LC3 and NBR-1, most preferred is LC3.

### Transcriptional Activity:

Transcription is the process of creating a complementary RNA copy of a sequence of DNA.
This process is the first step leading to protein biosynthesis and gene expression. The stretch of DNA transcribed into an RNA molecule encodes at least one gene. If the gene transcribed encodes a protein, the result of transcription is messenger RNA, which will then be used to create that protein via the process of translation [Lodish H. Berk, A. Zipursky S.L., Matsudeira P, Baltimore D., Darnell J. (2000). Molecular Cell Biology. fourth edition, W.H. Freeman and Company]. Alternatively, the transcribed gene may encode for either ribosomal RNA (rRNA) or transfer RNA (tRNA), other components of the protein-assembly process, or other ribozymes. Indicators for this parameter are analytes which change their expression level or posttranslational modifications such as phosphorylation or glycosylation in case the parameter is affected.
Examples for suitable analytes for transcriptional activity are TFIID, TFIIH, TFIIB, SP1, PTEFb (positive transcription elongation factor b), preferably RNA Pol II (pSer2/5), pan RNAPol II, Mcl-1, most preferred is RNA Pol II (pSer2/5).

### Protein Biosynthesis:

Protein biosynthesis is the term that describes the generation of protein using the genetic information of a cell. The three steps in protein synthesis are transcription, RNA processing, and translation. Translation (in molecular biological meaning) describes the stage of protein biosynthesis where messenger RNA produced by transcription is decoded by ribosomes (cellular protein synthesizing machines) to produce a specific amino acid chain, or polypeptide, that will fold into an active protein [Lodish H. Berk, A. Zipursky S.L., Matsudeira P, Baltimore D., Darnell J. (2000). Molecular Cell Biology. fourth edition, W.H. Freeman and Company]*.*
Indicators for this parameter are analytes which change their expression level or posttranslational modifications such as phosphorylation or glycosylation in case the parameter is affected.
Examples for suitable analytes for protein biosynthesis are PRKCA, MKNK1, MAPK1, mTOR, DIO2, Akt1, eIF4E, PPP1CA, MAPK3, eIF4G1, HSPB1, PTK2B, MKNK2, preferably eIF2alpha (pSer51), eIF4B (pSer422), 4E-BP1 (pThr37/46), most preferred is eIF4B (pSer422).

### Protein Secretion:

After protein biosynthesis most eukaryotic secretory proteins are secreted to the cell surface through a conventional secretory pathway. Such proteins contain aminoterminal or internal signal peptides that direct their sorting to the endoplasmic reticulum (ER). From the ER, proteins are transported to the extracellular space or the plasma membrane through the ER-Golgi secretory pathway [Osborne, A. R., Rapoport, T. A. & van den Berg, B. (2005). Protein translocation by the Sec61/SecY channel. Annu. Rev. Cell Dev. Biol. 21, 529-550 and Lee, M. C., Miller, E. A., Goldberg, J., Orci, L. & Schekman, R. (2004). Bi-directional protein transport between the ER and Golgi. Annu. Rev. Cell Dev. Biol. 20, 87-123]. In addition, cytoplasmic, nuclear and signal-peptide-containing proteins have been shown to reach the cell surface by non-conventional transport pathways [Nickel W. (2003). The mystery of nonclassical protein secretion. Eur. J. Biochem. 270, 2109-2119].
Indicators for this parameter are analytes which change their expression level or posttranslational modifications such as phosphorylation or glycosylation in case the parameter is affected.
Examples for suitable analytes for protein secretion are protein disulfide isomerase (PDI), Hsc70, calnexin, calreticulin, peptidyl-prolyl isomerase, N-acetylgalactosamine (GaINAc) transfer, preferred is CHOP.

### Mitochondrial Activity:

Mitochondria are organelles of eukaryotic cells and are the main site of adenosine triphoshate (ATP) production during aerobic metabolism. ATP is the most important molecule for capturing and transferring free energy in biological systems [McBride HM, Neuspiel M, Wasiak S (2006). Mitochondria: more than just a powerhouse. Curr. Biol. 16 (14): R551]. In addition to supplying cellular energy, mitochondria are involved in a range of other processes, such as signaling, cellular differentiation, cell death, as well as the control of the cell cycle and cell growth [Campbell, Neil A.; Brad Williamson; Robin J. Heyden (2006). Biology: Exploring Life. Boston, Massachusetts: Pearson Prentice Hall].
Indicators for this parameter are analytes which change their expression level or posttranslational modifications such as phosphorylation or glycosylation in case the parameter is affected.
Examples for suitable analytes for mitochondrial activity are enzymes of the Krebs cycle like Aconitase, Isocitrate dehydrogenase, Alpha-ketoglutarate dehydrogenase, Succinyl CoA synthetase, Succinic dehydrogenase, Fumarase, Maltate dehydrogenase, Citrate synthase or
Pyruvate dehydrogenase or preferably analytes like Complex I, II, III, IV, V, NNT and Frataxin, most preferred is Complex IV.

### Stress Response:

Throughout their lifetime, cells can be subject to a range of environmental sources of stress, including radiation, hypoxia, nutrient deprivation, free radicals and others, that can lead to irrevocable damage and cell death.
Accordingly, cells have evolved many different mechanisms to oppose these exogenous sources of stress. In addition there are endogenous sources of cellular stress. One such endogenous source of cellular stress arises in the endoplasmic reticulum (ER) of cells following the accumulation of misfolded proteins during protein synthesis (known as ER stress) [Todd DJ, Lee AH, Glimcher LH (2008). The endoplasmic reticulum stress response in immunity and autoimmunity. Nat Rev Immunol. (9):663-74]. An important group of proteins involved in stress responses are heat shock proteins. They have strong cytoprotective effects, are involved in many regulatory pathways, and behave as molecular chaperones for other cellular proteins [Kregel KC (2002). Molecular Biology of Thermoregulation Invited Review: Heat shock proteins: modifying factors in physiological stress responses and acquired thermotolerance. J Appl Physiol. (92): 2177-2186].
Indicators for this parameter are analytes which change their expression level or posttranslational modifications such as phosphorylation or glycosylation in case the parameter is affected.

Examples for suitable analytes for stress response are Hsp70, Hsp27, Hsp60, Hsp90, Hsp27 (pS78/82), most preferred is Hsp70.

### Oxidative Stress:

Oxidative stress represents an imbalance between the production and manifestation of reactive oxygen species (such as superoxide anion, hydrogen peroxide, hydroxyl radical and several others) and a biological system's ability to readily detoxify the reactive intermediates or to repair the resulting damage [Rada B, Leto TL (2008). "Oxidative innate immune defenses by Nox/Duox family NADPH oxidases" Contrib Microbiol 15: 164-87]. The effects vary. A cell can be able to overcome small perturbations and regain its original state. Strong hypoxia and more severe oxidative stress can cause cell death and even moderate oxidation can trigger apoptosis, while more intense stresses may cause necrosis [Lennon SV, Martin SJ, Cotter TG (1991). Dose-dependent induction of apoptosis in human tumour cell lines by widely diverging stimuli. Cell Prolif. 24 (2): 203-14].
Indicators for this parameter are analytes which change their expression level or posttranslational modifications such as phosphorylation or glycosylation in case the parameter is affected.

Examples for suitable analytes for oxidative stress are PRDX1 (peroxiredoxin 1), GPX1, ANXA1, CCND1, DERL2, ERN1, ERO1L, GPX1, GSK3B, HDAC1, HERPUD1, HMOX1, HSPA5, LONP1, PPP1R15A, PRDX2, PXN, SPHK1, TXNRD1, WFS1, preferably HIF-1alpha and Ho-1, most preferred is HIF-1alpha.

### Protein Degradation:

Cells have extra and intracellular pathways for degrading proteins. The life span of intracellular proteins varies from as short as a few minutes to as long as several years. Cells have several intracellular proteolytic pathways for degrading misfold or denaturated proteins, normal proteins whose concentration must be decreased, and foreign proteins taken up by the cell. One of the best understood cytosolic pathway is the ubiquitin-mediated protein degradation pathway which involves two steps: addition of a chain of a ubiquitin molecules to a target protein and proteolysis of the ubiquitin-labeled protein by a multisubunit complexes with several proteolytic activities called proteasomes [Lodish H. Berk, A. Zipursky S.L., Matsudeira P, Baltimore D., Darnell J. (2000). Molecular Cell Biology. fourth edition, W.H. Freeman and Company*].*
Indicators for this parameter are analytes which change their expression level or posttranslational modifications such as phosphorylation or glycosylation in case the parameter is affected.
Suitable analytes for protein degradation are all proteins which underly the proteasomal degradation pathway or are directly and indirectly involved in regulation of protein ubiquitinylation and protein degradation by the proteasomal machinery or are involved in assembly of the proteasomal complex, preferred is Polyubiquitin (Lys48).

It has been found that the parameters listed above are key parameters for the determination of the cell status including toxic effects or other side effects of potential drugs. In most cases not all 12 parameters need to be determined. Depending on the type of potential drug to be tested, its future application and its way of acting, it is often sufficient to only determine 4, preferably 5, more preferably between 6 and 9 of the 12 parameters. A person skilled in the art can find the suitable parameters for example by comparing the potential drug to be tested with other drugs or drug candidates with similar structure and/or mode of action that have already been tested and for which the suitable parameters are already known.

Beside the 12 parameters listed above, in some cases additional parameters can be included in the testing. Suitable additional parameters are metabolic parameters like amino acid metabolism, carbohydrate metabolism, lipid metabolism,vVitamin and cofactor metabolism, nucleotide metabolism, steroid metabolism or metabolism of mediators as well as other parameters like membrane docking, contact inhibition, organelle organization, establishment or maintenance of cell, polarity, cellular membrane organization, intermediate filament-based process, or cellular component movement. Examples of before-mentioned and additional parameters as well as examples for suitable analytes for those parameters are listed in table 1.

**Table 1**

| **Biological parameter** | **Potential analytes** |
|---|---|
| ribonucleoprotein complex biogenesis | TGS1 |
| establishment or maintenance of cell polarity | CDC42, RHOA, RAC1 |
| cellular membrane organization | DNM2, ARRB1,CLTA |
| intermediate filament-based process | VIM, SYNC, KRT15 |
| cellular component movement | ACTB, CFL1 |
| cell activation | TIMP1 |
| cell adhesion | FN1, ITGAV |
| microtubule-based process | TUBA1A |
| cytoplasm organization | KIF5B |
| gene silencing | SIRT2, DNMT1 |
| actin filament-based process | ELMO1 |
| cell projection organization | MYO1A |
| transmembrane transport | AQP1 |
| cell recognition | CADM1 |
| chromosome segregation | STAG1 |
| cell communication | CNTNAP1 |
| membrane biogenesis | EXOC4 |
| cell aging | PDCD4 |
| vesicle targeting | YKT6 |

### Examples of further suitable parameters are:

### Cellular membrane biogenesis and organization

The process by which a membrane is synthesized, aggregates, and bonds together.
A membrane is a single or double layer of lipid molecules that encloses all cells and many organelles (at least in eukaryotes) and includes associated proteins. Cellular Membrane organization is a process which results in the assembly and arrangement of constituent parts or disassembly of a membrane. The plasma membrane of mammalian cells is made of a combination of glycosphingolipids and protein receptors organized in glycolipoprotein microdomains termed lipid rafts. These specialized membrane microdomains compartmentalize cellular processes by serving as organizing centers for at least the assembly of signaling molecules, influencing membrane fluidity and membrane protein trafficking.

Thomas S., Pais A.P., Casares S and Brumeanu T.D. (2004). Analysis of lipid rafts in T cells. Molecular Immunology 41: 399-409.
Pike, L. J. (2008). "The challenge of lipid rafts". The Journal of Lipid Research 50: S323.

### Establishment or maintanance of cell polarity

Cell migration is a central process in the development and maintenance of multicellular organisms. Tissue formation during embryonic development, wound healing and immune responses all require the orchestrated movement of cells in particular directions to specific locations. Migrating cells have a polarity - a front and a back - which is essential for migration in a defined direction. In chemotaxing cells (move along a chemical concentration gradient), the stability of the front appears enhanced as the cell advances towards a higher concentration of the stimulating chemical. This polarity is reflected at a molecular level by a restriction of certain molecules to particular regions of the inner cell surface: thus the phospholipid PIP3 and activated Rac and CDC42 are found at the front of the cell, whereas Rho GTPase and PTEN are found towards the rear.

Parent, C. A.; Devreotes, PN (1999). "A Cell's Sense of Direction". Science 284 (5415): 765-70.
Ridley, A. J.; Schwartz, MA; Burridge, K; Firtel, RA; Ginsberg, MH; Borisy, G; Parsons, JT; Horwitz, AR (2003). "Cell Migration: Integrating Signals from Front to Back". Science 302 (5651)

### Ribonucleoprotein complex biogenesis

The cellular process by which a complex containing RNA and proteins is synthesized, aggregates and bonds together. Removal of introns from eukaryotic nuclear messenger RNA precursors is catalysed by a large ribonucleoprotein complex called the splicosome which consists of small ribonucleoprotein particles and auxiliary protein factors. Ribonucleoproteins are nucleoproteins that contain RNA, i.e. it is an association that combines ribonucleic acid and protein together. A few known examples include the ribosome, the enzyme telomerase, vault ribonucleoproteins, and small nuclear ribonucleoproteins (snRNPs), which are implicated in pre-mRNA splicing and are among the main components of the nucleolus.

Genetic evidence for base pairing between U2 and U6 snRNA in mammalian mRNA splicing. Datta, B., Weiner, A.M. Nature (1991)

### Intermediate filament-based and microtubule- based process

Any cellular process that depends upon or alters the intermediate filament cytoskeleton and/or the microtubule cytoskeleton and their associated proteins. The cytoskeleton is a cytoplasmic system of fibers which is essential for cell motility. It consists of three types of cytosolic fibers: microfilaments, intermediate filaments and microtubules. The fibers are well-ordered polymers built from small protein subunits.

Lodish H. Berk, A. Zipursky S.L., Matsudeira P, Baltimore D., Darnell J. (2000). Molecular Cell Biology. fourth edition, W.H. Freeman and Company*].*

### Cellular component movement

The directed, self-propelled movement of a cellular component without the involvement of an external agent such as a transporter or a pore. In some cases actin and/or actin-binding proteins are involved in such movement.

Lodish H. Berk, A. Zipursky S.L., Matsudeira P, Baltimore D., Darnell J. (2000). Molecular Cell Biology. fourth edition, W.H. Freeman and Company*].*

### Actin filament-based process

Any cellular process that depends upon or alters the actin cytoskeleton, that part of the cytoskeleton comprising actin filaments and their associated proteins.

Lodish H. Berk, A. Zipursky S.L., Matsudeira P, Baltimore D., Darnell J. (2000). Molecular Cell Biology. fourth edition, W.H. Freeman and Company*].*

### Cytoplasm organisation

A process that is carried out at the cellular level which results in the assembly, arrangement of constituent parts, or disassembly of the cytoplasm. The cytoplasm is all of the contents of a cell excluding the plasma membrane and nucleus, but including other subcellular structures.

Lodish H. Berk, A. Zipursky S.L., Matsudeira P, Baltimore D., Darnell J. (2000). Molecular Cell Biology. fourth edition, W.H. Freeman and Company*].*

### Cell activation

A change in the morphology or behavior of a cell resulting from exposure to an activating factor such as a cellular or soluble ligand. For example activation of the cells of the immune system (T cell activation, B cell activation or activation of dendritic cells).

Murphy K., Travers P., Walport M. (2008). Janeway's immunobiology. Seventh edition, Garland Science, Tylor & Francis Group, LLC

### Cell adhesion

Adhesion of cells is a primary feature of the architecture of many tissues. A huge number of cell surface proteins mediate adhesion. There are at least 5 classes of cell surface proteins: cadherins, immunoglobulin superfamily, selectins, mucins (all mediate cell to cell adhesion and integrins (mediate cell to matrix adhesion).

Lodish H. Berk, A. Zipursky S.L., Matsudeira P, Baltimore D., Darnell J. (2000). Molecular Cell Biology. fourth edition, W.H. Freeman and Company*].*

### Cell recognition

The process by which a cell in a multicellular organism interprets its surroundings (at least neuron recognition, phagocytosis recognition, cell-cell recognition, cell matrix recognition).
Lodish H. Berk, A. Zipursky S.L., Matsudeira P, Baltimore D., Darnell J. (2000). Molecular Cell Biology. fourth edition, W.H. Freeman and Company*].*
Murphy K., Travers P., Walport M. (2008). Janeway's immunobiology. Seventh edition, Garland Science, Tylor & Francis Group, LLC

### Cell communication

Any process that mediates interactions between a cell and its surroundings. Encompasses interactions such as signaling or attachment between one cell and another cell, between a cell and an extracellular matrix, or between a cell and any other aspect of its environment.

Lodish H. Berk, A. Zipursky S.L., Matsudeira P, Baltimore D., Darnell J. (2000). Molecular Cell Biology. fourth edition, W.H. Freeman and Company]*.*

### Gene silencing

Any transcriptional or post-transcriptional process carried out at the cellular level that results in long-term gene inactivation. Gene silencing is a general term describing epigenetic processes of gene regulation. The term gene silencing is generally used to describe the "switching off' of a gene by a mechanism other than genetic modification.

Zaratiegui M, Irvine DV, Martienssen RA (2007) Noncoding RNAs and gene silencing. Cell 128(4):763-76.

### Cell projection organization

A process that is carried out at the cellular level which results in the assembly, arrangement of constituent parts, or disassembly of a prolongation or process extending from a cell, e.g. a flagellum or axon.
Lodish H. Berk, A. Zipursky S.L., Matsudeira P, Baltimore D., Darnell J. (2000). Molecular Cell Biology. fourth edition, W.H. Freeman and Company*].*

### Transmembrane transport

The process whereby a solute is transported from one side of a membrane to the other. Facilitated diffusion is the spontaneous passage of molecules or ions across a biological membrane passing through specific transmembrane integral proteins. A membrane transport protein is a membrane protein involved in the movement of ions, small molecules, or macromolecules, such as another protein across a biological membrane. Transport proteins are integral membrane proteins. They exist within and span the membrane across which they transport substances. The proteins may assist in the movement of substances by facilitated diffusion or active transport. These mechanisms of action are known as carrier-mediated transport.
Pratt, Charlotte Amerley; Voet, Donald; Voet, Judith G. (2002). Fundamentals of biochemistry upgrade. New York: Wiley. pp. 264-266.

### Chromosome segregation

The process by which genetic material, in the form of chromosomes, is organized into specific structures and then physically separated and apportioned to two or more sets. Chromosome segregation begins with the alignment of chromosomes at the metaphase plate, includes chromosome separation, and ends when chromosomes have completed movement to the spindle poles. In mitosis, a complete copy of each one is made. In meiosis, one chromosome from each pair migrates to opposite ends of the cell and the genes are split to make a gamete.
Lodish H. Berk, A. Zipursky S.L., Matsudeira P, Baltimore D., Darnell J. (2000). Molecular Cell Biology. fourth edition, W.H. Freeman and Company*].*

### Cell aging

Progression of the cell from its inception to the end of its lifespan. One hypothesis proposes that ageing is caused by accumulating genotoxic stress provoked by the progressive loss of telomeres, which leads to replicative senescence and apoptosis, whereas another postulates that ageing is the result of progressive mitochondrial malfunction.

Kirkwood, T. B. Understanding the odd science of aging (2005). Cell 120, 437-447
Balaban, R. S., Nemoto, S. & Finkel, T (2005). Mitochondria, oxidants, and aging. Cell 120, 483-495

### Vesicle transport

The process by which vesicles are directed to specific destination membranes. Targeting involves coordinated interactions among cytoskeletal elements (microtubules or actin filaments), motor proteins, molecules at the vesicle membrane and target membrane surfaces, and vesicle cargo.

Lodish H. Berk, A. Zipursky S.L., Matsudeira P, Baltimore D., Darnell J. (2000). Molecular Cell Biology. fourth edition, W.H. Freeman and Company]*.*

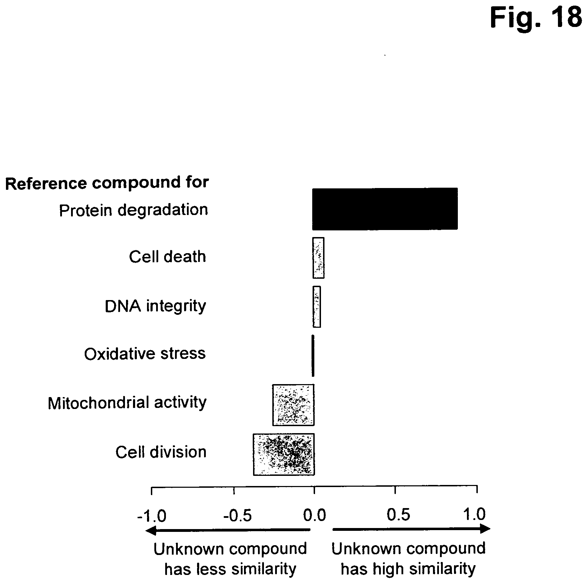

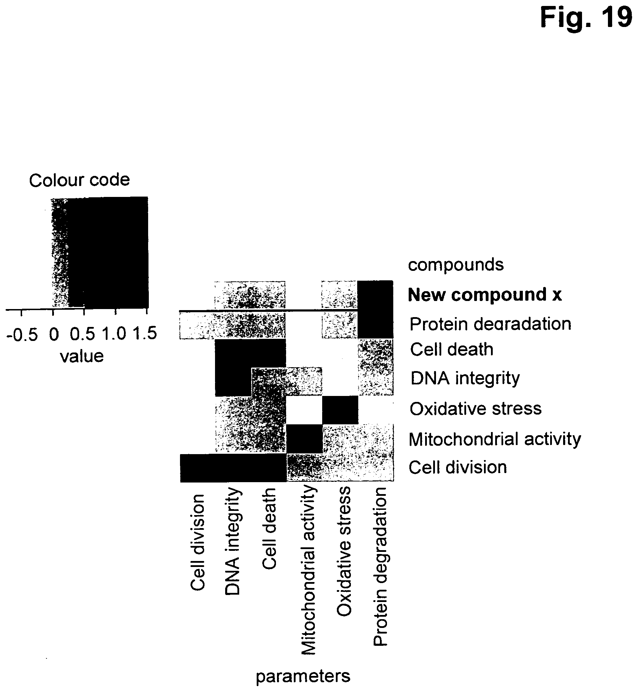

When performing the method according to the present invention one gets a set of data comprising the results of the analyses that have been performed for one potential drug. These results may comprise analyses for different parameters as well as analyses with different analytes (for one parameter), analyses with different cells or results obtained under different experimental conditions (time, temperature, concentration of reagents etc.) and/or using different assay formats. All experiments that have been performed with one potential drug and all results obtained with these experiments give the set of data for one potential drug. As a consequence, for each potential drug that is tested with the method according to the present invention, one obtains a set of data. Each result of one single experiment (a single experiment being an analysis performed with one potential drug using a certain type of cells, testing for one certain analyte and certain experimental conditions) being one data point in the set of data. Preferably, all data points and all sets of data are stored in a database. The database preferably allows for sorting of the data points according to certain characteristics of the experiments with which the data points have been generated, like potential drug, type of cell, analyte, experimental conditions etc.. This offers the possibility to compare sets of data or data points with each other. For example, if for a potential drug 1, data points have been generated for the parameter Oxidative stress using the analyte HIF-1alpha and DNA integrity using the analyte H2A.X (pSer139) under certain experimental conditions using a certain cell line, these data points could be compared with those obtained for a potential drug 2 under the same experimental conditions using the same cell line and the same analytes. This offers the possibility to compare the mode of action of different potential drugs and to find similarities and differences. In addition, the sets of data of potential drugs can be compared with sets of data obtained under comparable conditions for known drugs having a known mode of action. Also dose-response curves can be established for each potential drug by contacting the cells with successively increasing doses of the potential drug. The data base will preferably comprise more than 50 preferably more than 100 reference data sets which have been generated by the method according to the present invention. Examples of possible data processing and visualization methods are shown in Figures 18 and 19.
To generate the data that are processed and visualized in Figures 18 and 19, an exemplary data set comprising 8 single assays and 10 compounds was analyzed at 1 concentration and 4 different time points: For each specific combination of compound/assay/time point a difference D was derived by subtracting the signals of the treated samples from the matching signals of the controls. Assay-specific normalization factors N were derived as the sum of D's across the time courses for the assay-associated reference compounds. A summary value S for each compound/assay combination was calculated by summation of D's for the time course signals of each single compound. Normalized data Z was then derived by dividing the summarized values S by the normalizing factor N. The figures show two ways of visualizing the similarities in across-assay profiles induced by a new compound X and our reference compounds. Figure 18: The generic similarity index (Pearson correlation of across-assay Z profiles of reference compounds and query compounds) shows that the unknown compound which was analyzed here has high similarity (dark grey bar) to the reference compound which affects protein degradation (Pearson correlation: dark grey stands for strong positive correlation, light grey means weak positive or even anti-correlation), suggesting that the new compound influences this cellular process. Figure 19: The heatmap visualizes the profiles of normalized signals induced by a new compound with those profiles induced by reference compounds. The reference compounds that are used should have a known effect. For example the compounds used in the setup visualized in Figure 19 are known have effects on protein degradation (compound in second line), cell death (compound in third line), DNA integrity (compound in third line), oxidative stress (compound in fourth line), mitchondrial activity (compound in fifth line) and cell division (compound in sixth line). The columns of the map show the results for the parameters that have been tested. The parameters are indicated below the map. The analytes that have been tested are histone H3(pSer10) for Cell division, histone gH2A.X for DNA integrity, cleaved Parp (Asp214) for Cell death, Complex IV for Mitochondrial Activity, Hif-1a for Oxidative Stress and Polyubiquitin (Lys48 linkage) for Protein degradation.
As a result, the map shows that the unknown compound shows a profile that is very similar to the compound in the second line that is know to have an effect on protein degradation.

Further disclosed is an assay format comprising means for determining at least 4 parameters out of the following group of parameters:
DNA Integrity
Cell Division
Cell Death
Transcriptional Activity
Protein Biosynthesis
Mitochondrial Activity
Stress Response
Oxidative Stress
Protein Degradation
Protein Secretion
Autophagy
Metabolic Activity

The assay format is typically a kit of parts at least comprising the capture groups for determining at least 4 parameters out of the group of the inventive parameters. The capture groups preferably are antibodies which are able to bind to one of the analytes.

In a preferred embodiment, the assay format additionally comprises detection antibodies which are able to bind to the capture groups or the analyte and are further able to generate a detectable signal. Examples of suitable detection antibodies are fluorescently labelled antibodies or HRP-conjugated antibodies.

In a preferred embodiment, the assay is in a multiplexed format. In a very preferred embodiment the assay format is bead-based. Especially preferred it comprises beads which are colour coded with at least two different fluorescent dyes. In this assay format, the capture group (preferably an antibody) specific for an analyte is coupled to one type of fluorescent-labeled beads. Preferably the assay format not only comprises the capture groups coupled to the beads but also a second antibody which can also bind to the analyte and which directly or indirectly produces a detection signal (sandwich principle).
In a preferred embodiment, the assay format comprises at least
- a capture bead mix or single capture beads
- a detection antibody mix or single detection antibodies
- one or more of the following reagents: cell extraction reagent (lysis buffer), phosphatase inhibitor mix, protease inhibitor mix, benzonase

The assay format may also comprise
- at least one Assay running buffer to perform the measurement
- washing buffer
- label in case indirect label method is used
- standard (e.g. recombinant protein or peptide or cell lysate (eg. Phosphatase treated or untreated, or compound treated or untreated)
   - reaction devices (e.g. 96 well plates)
   - reference compounds
   - software + reference database (e.g comprising data from about 100 compounds)

The above disclosure generally describes the present invention. A more thorough understanding can be obtained by reference to the following specific examples which are provided herein for purposes of illustration only and are not intended to limit the scope of the invention. All documents cited above or below and of corresponding EP application EP 11007865.6, filed September 28, 2011 are included by reference.

### Examples

### Methods

### Reference inhibitor treatment and cell lysis

HepG2 and HEK293 cells are cultured at 37°C and 5%CO₂ until 80% confluence. For inhibitor treatment cells are cultured in sixwells (HepG2 7.6 - 10 x10⁵ cells per well, Hek293 1- 8 x 10⁵ cells per well depending on culture times). After 12h cells are treated with reference inhibitors like Bleomycin (10⁻⁴ - 10⁻⁸ M for 6-48 h), Taxol (10⁻⁷ - 10⁻¹¹ M for 6-48 h), Camptothecin (10⁻⁵ - 10⁻⁹ M for 6-48 h), DRB (5,6-Dichlorobenzimidazole Riboside 10⁻⁴ - 10⁻⁷ M for 6-48 h), Trichostatin A (10⁻⁴ - 10⁻⁷ M for 6-48 h), Actinomycin D (10⁻⁴ 10⁻⁷ M for 6-48 h), Rotenon (10⁻⁴ - 10⁻⁸ M for 48-96h), Antimycin (10⁻⁴ 10⁻⁸ M for 48-96h), Azide (10⁻⁴ - 10⁻⁸ M for 48-96h), Oligomycin (10⁻⁴ - 10⁻⁸ M for 48-96h), 2,4 DNP (10⁻⁴ - 10⁻⁸ M for 48-96h), Geldanamycin (10⁻⁶ - 10⁻¹⁰ M for 6-48h), 17-AAG (10⁻⁶ -10⁻¹⁰ M for 6-48h), 17-DMAG (10⁻⁶ - 10⁻¹⁰ M for 6-48h), MG132 (10⁻⁶ - 10⁻¹⁰ M for 6-48h), Velcade (10⁻⁶ - 10⁻¹⁰ M for 6-48h), Deferoxamin (10⁻⁴ - 10⁻⁸ M for 6-48h), CoCl₂ (10⁻⁴ - 10⁻⁸ M for 6-48h), DMOG (Dimethyloxalylglycin, 10⁻⁴ - 10⁻⁸ M for 6-48h), Thapsigargin (10⁻⁶ - 10⁻⁹ M for 6-48h), Tunicamycin (10⁻⁶ - 10⁻⁹ M for 6-48h), Brefeldin (10⁻⁶ - 10⁻⁹ M for 6-48h), Bafilomycin (10⁻⁷ - 10⁻¹² M for 6-48h), respectively. As a positive control vehicle control cells are treated in parallel with the respective solvent of the inhibitor e.g. DMSO, H₂O, Methanol or Ethanol.

For cell lysis cell culture supernatant is removed and cells are washed with TBS (Tris buffered saline). Cells are incubated either with 0.2-2ml NP40 cell lysis buffer (20mM Tris pH 7.4, 150 mM NaCl, 1% NP40, 10% Glycerol) or RIPA lysis buffer (10 mM NaH₂PO₄/ Na₂HPO₄ pH 7.2, 2 mM EDTA, 1% Triton-X 100, 0.1% SDS, 0.5% Na-Deoxycholate, 10% Glycerol), Tween20 lysis buffer (1% Tween 20, 0,5% Na-Deoxycholat, 10 mM PIPES), Maltoside lysis buffer (25mM HEPES pH 7.5, 100 mM NaCl, 29 mM Dodecyl-Maltoside) or maltoside lysis buffer with high concentration of salt (25mM HEPES pH 7.5, 350 mM sodium chloride, 29 mM Dodecyl-Maltoside, 1 mM magnesium chloride). Supplements are always added to cell lysis buffer immediately before use: Protease inhibitor cocktail 1:1000 (EMD Chemicals), Phosphatase Inhibitor Cocktail V 1:50 (EMD Chemicals) and Benzonase 0.0025 to 0.25 units/µl (EMD Chemicals). Cell lysates are incubated on ice for 20 to 30 min and cleared by filtration (0.45 µm pore size) or by centrifugation for 5min with 10000 x g at 4°C. To determine total protein concentrations a BCA protein assay is performed.

For some experiments histone proteins are extracted using acid extraction of a nuclear fraction. Attached cells are washed with phosphate-buffered saline solution, lysed in NETN buffer (20 mM Tris pH7.4, 0.15 M NaCl, 2.5 mM sodiumorthovanadate, 1 mM EDTA, 0.5% Igepal), and scraped off the plate. The lysates are transferred to 1.5 ml polypropylene tube and incubated on ice for 5 min. The lysates are centrifuged at 10.000 x g for 5 min at 4°C and the supernatant fraction is collected in a fresh tube. 0.1 M Hydrochloride is added to the pellet fraction, vortexed and incubated on ice for additional 5 min. After additional centrifugation the supernatant is collected, neutralized with an equal volume of 0.1 M Tris-HCl buffer pH 7.4. The acid-extracted lysates are subsequently denatured in SDS sample buffer und analysed by SDS-PAGE.

For one-step extraction of nuclear proteins together with cytoplasmatic proteins the cells are lysed using maltoside lysis buffer with high concentration of salt with supplements as described above (benzonase 0.25 units/µl).

### Western blot Analysis

Proteins of HepG2 and HEK293 cell lysates are separated according by SDS-PAGE (sodium dodecyl sulfate-polyacrylamide gel elecrophoresis). In brief: 10-50 microgram of cell lysate is mixed with SDS-PAGE sample buffer and heated at 95°C for 5 min. Samples are loaded on a 4-12% Bis-Tris gradient gel. The gel is run in a commercial electrophoresis chamber (using MOPS buffer as running buffer) at 70 milliampere for 90 min. Separated proteins are then blotted onto a nitrocellulose or PFDF membrane (Merck Millipore) using Tris-Glycine buffer containing 10% methanol. Blotting is performed at 100 volt for 60 min.

After protein blotting membranes are blocked with either Odyssey-Blocking buffer as indicated by the manufacturer (LI-COR) or with 5% BSA (in TBS buffer containing 0.1 % Tween20) at room temperature for 1 hour. Membranes are then incubated with unlabelled primary antibody specific at 4°C over night. Detection is performed either as indicated by the manufacturer (Odyssey infrared imaging system using fluorescently labelled secondary antibodies) or by chemiluminescence detection using a HRP-conjugated secondary antibodies as indicated by the manufacturer. For chemiluminescence detection membranes are washed four times with TBS buffer (containing 0.1% Tween20) for 5 minutes per washing. Then membranes are incubated with a commercial HRP-conjugated secondary antibody at room temperature for 60 minutes. Membranes are washed four times as mentioned above and incubated with a commercial enhanced chemoluminescence solution (as indicated by the manufacturer). In several cases densitometric quantification is performed using commercially available software (Odyssey® application software version 3.0, LI-COR® Biosciences; QuantityOne, Bio-Rad Laboratories)

### ELISA (Enzyme linked immunosorbent assays)

Detection of γH2A.X is performed using a cell-based ELISA assay (Merck Millipore, #17-327). In brief, HEK293 or HepG2 cells are plated at a density of 50.000 cells in a 96-well tissue culture plate and cultivated overnight. On the next day, cells are stimulated for 6 hours with the DNA damaging drug Bleomycin at different concentrations. After stimulation the cells are fixed for 7 min in 95% ethanol/5% acetic acid and subsequently for 5 min with 1% formaldehyde in Tris-buffered saline (TBS). Quenching is performed using TBS containing 1% H₂O₂ for 20 minutes. Wells are blocked with blocking buffer (TBS containing 3% bovine serum albumin) for one hour.

The wells are washed three times with TBS and then incubated at 4° Celsius overnight with mouse monoclonal antibody diluted at 1:8000 in blocking buffer. On the next day, wells are washed three times with TBS and incubated with anti-mouse antibody coupled with horseradish peroxidase diluted in blocking buffer. After three final washes with TBS the chemiluminescence reagent is added. Chemiluminescence is measured after 10 min incubation with a Mithras Multilabel reader (Berthold). Detection of phospho histone H3 (p-Ser10) is performed using a PathScan ELISA system from Cell Signaling Technologies. Cell lysates prepared from HEK293 or HepG2 cells treated for 24 hours with different concentrations of Taxol or DMSO vehicle alone are incubated in microwell strips at 4° Celsius overnight. On the next day, cavities of the wells are washed four times with washing buffer and the secondary antibody solution is added. One hour later the wells are washed four times with washing buffer and incubated with a detection antibody coupled with horseradish peroxidase. The wells are washed four times with washing buffer and TMB substrate solution is added. After 30 min stop solution is added and the absorption at 450 nm is measured using a spectrophotometer of standard laboratory practice (e.g. Tecan Sunrise).

### Luminex xMAP® Assay

xMAP® beads are coupled with specific capture antibodies as described by the manufacturer (Luminex Inc.). 3000 beads per well and per analyte are added to a 96well filter plate and are incubated with cell lysate over night at 4°C (10µg total protein per well). For a multiplex approach two up to 500 antibody coupled bead reagions can be measured in parallel. After three wash steps with WideScreen® Assay Diluent parameters are detected with a biotinylated detection antibody (1 h agitation at room temperature) and phycoerythrin-conjugated Streptavidin (45 min agitation at room temperature). After three wash steps and resuspension of beads with 120µl WideScreen® Assay Diluent Microspheres are analysed in a Luminex¹⁰⁰ machine as described by the manufacturer.

### Data Analysis

Raw data generated via different techniques (Luminex, luminescence, absorption spectroscopy data, and densitometric/fluorimetric quantitation of western blots) are processed in a similar manner. If applicable, background values are subtracted from data and data are converted to % of control (vehicle treated control was set to 100% if possible).

### Exemplary analysis of analytes for the 12 preferred parameters

### Optimal one-step nuclear protein extraction.

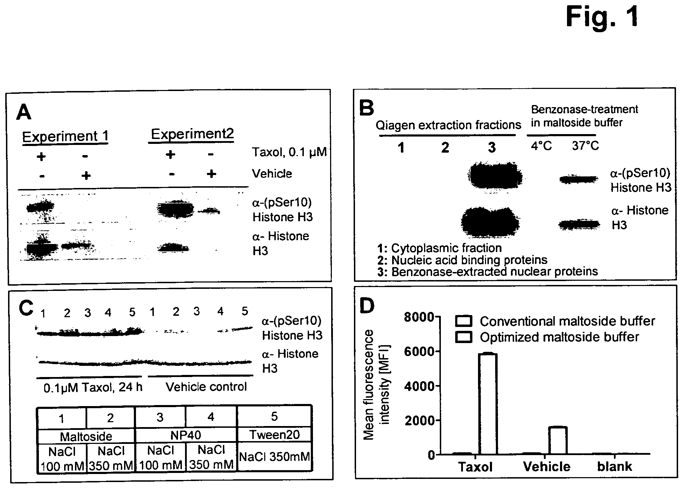

Hep G2 cells were treated with Taxol or vehicle control (DMSO) and lysed after 24 h using several lysis conditions. Phospho-histone H3 (pSer10) and total histone H3 were detected by Western Blot (A-C) or Luminex (D). The results of the experiments are shown in Figure 1: **A**. cells were lysed using conventional maltoside buffer with benzonase (0.25 units/µl). **B**. Vehicle-treated cells were lysed using either the multi-step Qproteome protein fraction extraction kit (Qiagen) as described by the manufacturer or by an one-step conventional maltoside buffer lysis with benzonase (0.25 units/µl) at different temperatures (4 °C and 37°C). Whereas histone H3 and phospho-histone H3 can be found in the nuclear fraction of the Qproteome kit, H3 and phospho-H3 were not extractable at 4 °C and only partly extractable at 37°C using a one-step lysis with conventional maltoside buffer. C. One-step cell lysis was performed at 4 °C using maltoside, NP40 or Tween 20 buffer with high concentration of sodium chloride (50 - 500 mM, preferred are 350 mM) and benzonase (0.25 units/µl). Phosphorylated as well as total histone H3 can be extracted by adding high concentration of benzonase and salt to the buffer system. D. Phospho-histone H3 (pSer10) can be optimally detected in cells which have been lysed with one-step lysis at 4°C using maltoside lysis buffer with high concentration of salt (350 mM sodium chloride, 1 mM magnesium chloride) and benzonase (0.25 units/µl).

### DNA Integrity, analyte: γH2A.X

Rationale: Induction of phosphorylation on serine 139 upon DNA damage is a consequence of various genotoxic agents.

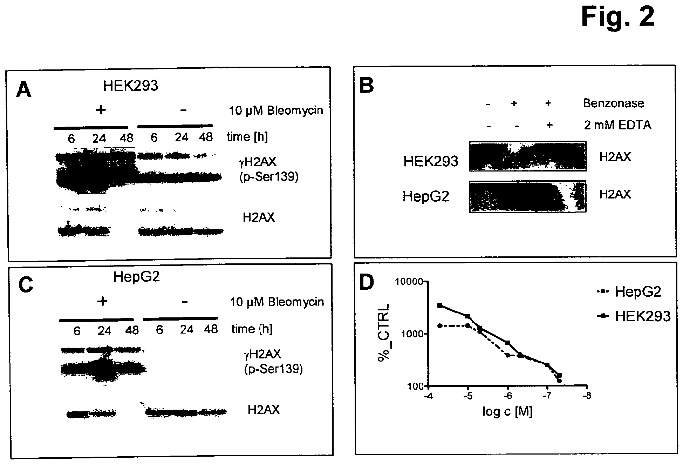

Experiments are performed according to the methods given above. The results of the experiments are shown in Figure 2:
HEK293 (A,B/upper figure,D/straight line) or HepG2 (B/lower figure,C,D/dotted line) cells were cultured at 37°C, 5%CO₂ and treated with Bleomycin or DMSO as vehicle control at the indicated concentrations and for the indicated time points. **A, C: Time dependence of γH2A.X levels after treatment with 10 µM Bleomycin**. Acid-extracted fractions from cell lysates were analyzed by Western Bot analysis using γH2A.X(p-Ser139) and H2A.X-specific antibodies. **B: Efficient extraction of H2A.X using benzonase.** Cell lysates from untreated cells were prepared with maltoside-detergent containing lysis buffer in the absence or presence of benzonase or benzonase plus the inhibitor EDTA. **D: Dose-dependence of γH2A.X (p-Ser139) at different concentrations of Bleomycin**. Bleomycin was incubated for 6 hours with the cells, the cells were fixed and permeabilised and γH2A.X (p-Ser139) was determined using a specific cell-based ELISA assay. Data are shown as % of DMSO control.

### Cell Division, analyte: Histone H3 (pSer10)

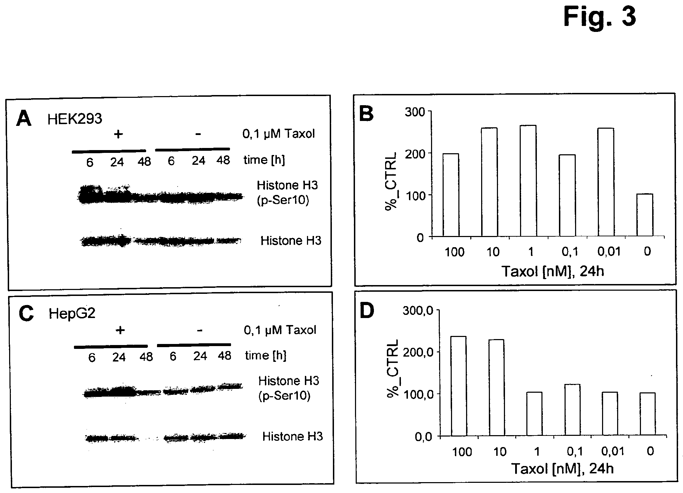

Rationale: Marker for mitotic fraction of cells. Cell cycle arrest either indicated by reduction or increase on Histone H3 (pSer10) content. Experiments are performed according to the methods given above. The results of the experiments are shown in Figure 3:
HEK293 (A, B) or HepG2 (C, D) cells were cultured at 37°C, 5%CO₂ and treated with Taxol or DMSO as vehicle control at the indicated concentrations and for the indicated time points. **A, C: Time-dependent change in phospho-histone H3 (p-Ser10) levels**. Acid-extracted fractions from cell lysates were analyzed by Western Blot analysis using phospho-histone H3 (p-Ser10) and histone H3-specific antibodies. **B, D: Dose-dependence of phospho-histone H3 (pSer-10) at different concentrations of Taxol.** Taxol was incubated for 24 hours with the cells at the indicated concentrations, cells were lysed and p-histone H3 (p-Ser10) was determined using a specific ELISA assay. Data are shown as % of DMSO control.

### Cell Death, analyte: cleaved PARP

Rationale: PARP is cleaved upon execution of apoptosis by activated effector caspases like caspase-3 and caspase-7.

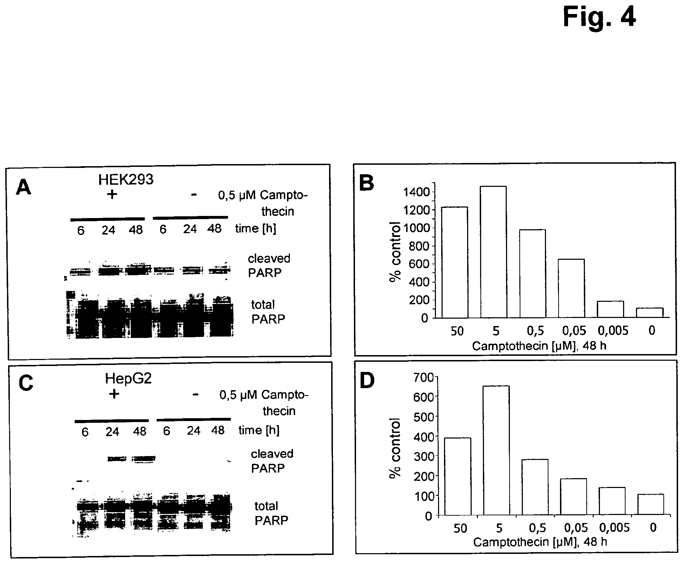

Experiments are performed according to the methods given above. The results of the experiments are shown in Figure 4:
HEK293 (A, B) or HepG2 (C, D) cells were cultured at 37°C, 5%CO₂ and treated with Campthothecin or DMSO as vehicle control at the indicated concentrations and for the indicated time points. A, C: Time-dependent change in PARP cleavage (cleaved PARP). Cell lysates were analyzed by Western Blot analysis using antibodies specifically detecting cleaved PARP and total PARP. B, D: Dose-dependence of cleaved PARP after treatment with different concentrations of Camptothecin. Camptothecin was incubated for 48 hours with the cells at the indicated concentrations, cells were lysed and cleaved PARP was determined by Western Blotting. After densitometric quantification of Western Blots the data were transformed to % of DMSO control.

### Transcriptional Activity, analyte: RNA Pol II (pSer 2/5)

Rationale: DNA-dependent RNA Polymarase II (RNA Pol II) gets phosphorylated at serine 2/5 when transcription is activated. Transcription inhibition decreases phosphorylation of RNA Pol II.

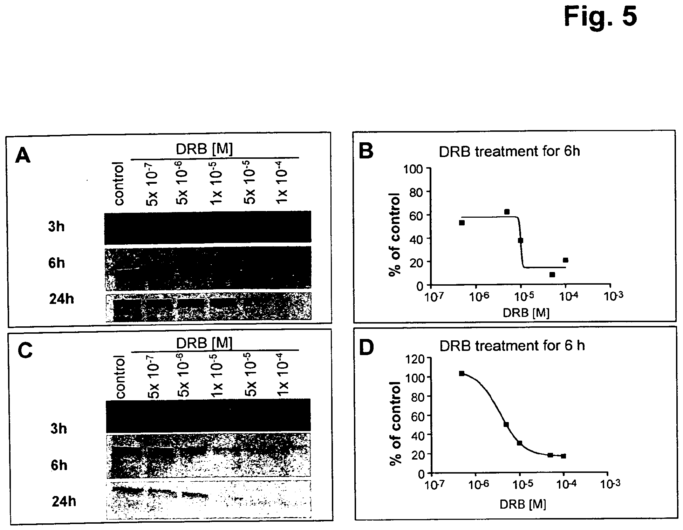

Experiments are performed according to the methods given above. The results of the experiments are shown in Figure 5:
HEK293 (A, B) or HepG2 (C, D) cells were cultured at 37°C, 5% CO2 and treated with 5,6-Dichlorobenzimidazole riboside (DRB) or DMSO as vehicle control at the indicated concentrations and for the indicated time points.

A,C: Time and concentration-dependent change of RNA pol II phosphorylation (pSer2/5) was detected in cell lysates by Western Blot technique. Antibodies specifically detecting RNA Pol II (pSer2/5) or total RNA Pol II and fluorescence imaging (Odyssey, LI-COR) were used. B,D:
Densitometric quantification of Western Blot signals from A and C was performed. Data are depicted as percentage of control.

### Protein Biosynthesis, analyte: elF4B (pSer422)

Rationale: elF4B (pSer422) is recruited to pre-initiation complex and enhances the activity of RNA helicase. Translation inhibition increases elF4B (pSer422) (positive feedback loop).

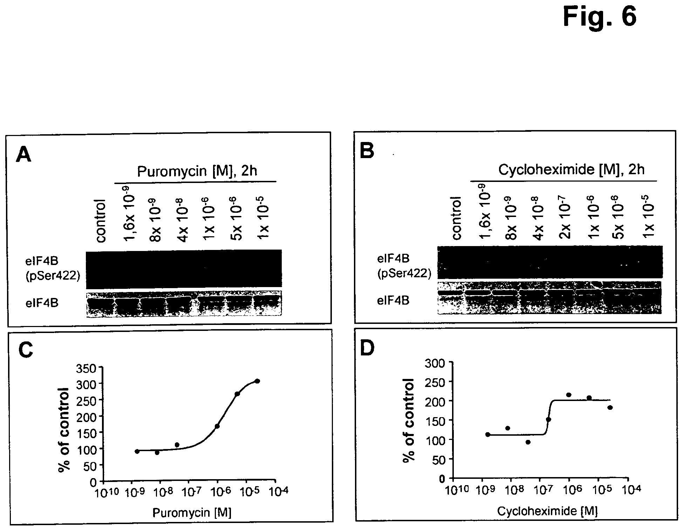

Experiments are performed according to the methods given above. The results of the experiments are shown in Figure 6:
HepG2 cells were cultured at 37°C, 5%CO₂ and treated with Puromycin or H₂O as control (A,C) or with Cycloheximide and EtOH as control (B,D) at the indicated concentrations for two hours. A, B: Concentration-dependent change in elF4B phosphorylation at serine 422 (pSer422). Cell lysates were analyzed by Western Blot analysis using antibodies specifically detecting elF4B (pSer422) and total elF4B. For analysis fluorescence imaging (Odyssey, LI-COR) was used C,D: Densitometric quantification of Western Blot signals from A and B was performed. Data are depicted as percentage of control.

### Mitochondrial Activity, analyte: Complex IV

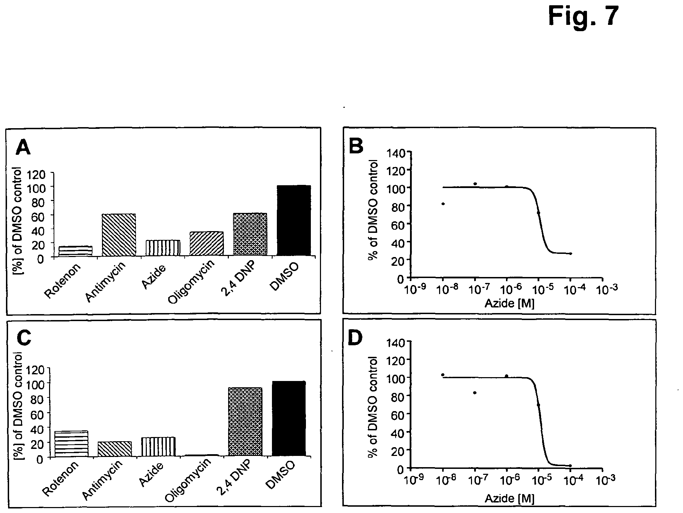

Rational: Complex IV is partially transcriped from mitochondrial DNA. Lower Complex IV expression levels indicate mitochondrial damage. Experiments are performed according to the methods given above. The results of the experiments are shown in Figure 7:
Hek293 (A+B) or HepG2 (C+D) cells were cultured at 37°C, 5%CO₂ and treated with 10µM Rotenon, Antimycin, Azide, Oligomycin, 2,4 DNP or with DMSO as a vehicle control for 96h (A+C) or 72h (B+C) with Azide in a concentration dependend manner. Cell lysates were analyzed with the WideScreen® Oxidative Phosphorylation Panel (Merck Millipore) to determine Complex IV expression levels. Data are shown as % of DMSO control.

### Stress Response, analyte: HSP70

Rational: Induction of HSP70 expression indicates stress response upon HSP90 inhibition.

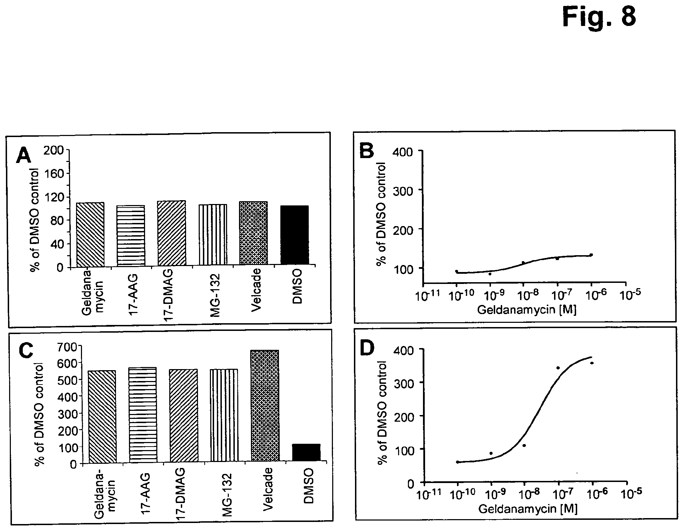

Experiments are performed according to the methods given above. The results of the experiments are shown in Figure 8:
Hek293 (A+B) or HepG2 (C+D) cells were cultured at 37°C, 5%CO₂ and treated with 1µM Geldanamycin, 17-AAG, 17-DMAG (all HSP90 inhibitors), MG132, Velcade (Proteasome inhibitors) or with DMSO as a vehicle control for 24h (A+C) or 48h (B+C) with Geldanamycin in a concentration dependend manner. Cell lysates were analyzed with the WideScreen® Heat Shock Protein Panel (Merck Millipore) to determine HSP70 expression levels (A-D). Data are shown as % of DMSO control.

### Oxidative Stress, analyte: HIF-1α

Rational: Induction of HIF-1α expression is shown as a master regulator of the cellular response to hypoxia (reduced oxygen pressure).

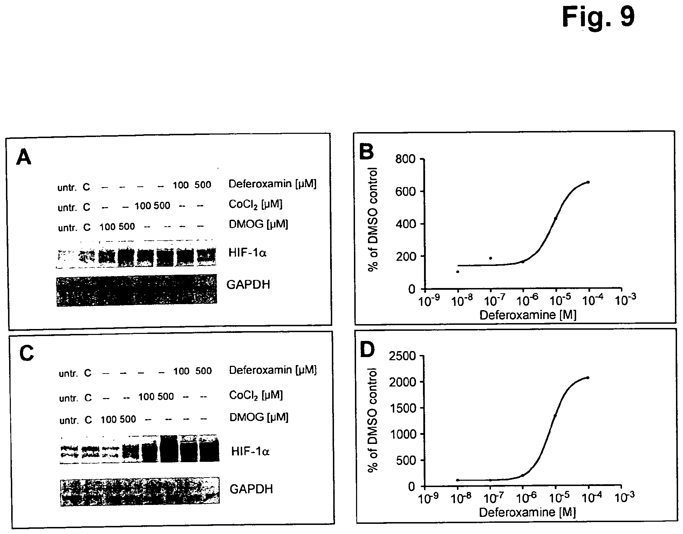

Experiments are performed according to the methods given above. The results of the experiments are shown in Figure 9:
Hek293 (A+B) or HepG2 (C+D) cells were cultured at 37°C, 5%CO2 and treated with 100µM or 500µM Deferoxamin, CoCl2, and Dimethyloxalylglycin (DMOG; Fe3+ chelator and hypoxia induction) or with DMSO as a vehicle control for 24h (**A+C**) or 6h (**B+C**) with Deferoxamine in a concentration dependend manner. Cell lysates were analyzed with Western Blot analysis (A+C) or an Hif-1a specific Luminex® xMAP® assay (**B+D**) to determine Hif-1a expression levels. Data are shown as % of DMSO control.

### Protein Degradation, analyte: Polyubiquitin (Lys48)

Rational: Proteins are polyubiquitinylated via Lys48 binding when destinated for proteasomal degradation. Accumulation of Polyubiquitin (Lys48) indicates inhibition of degradation.

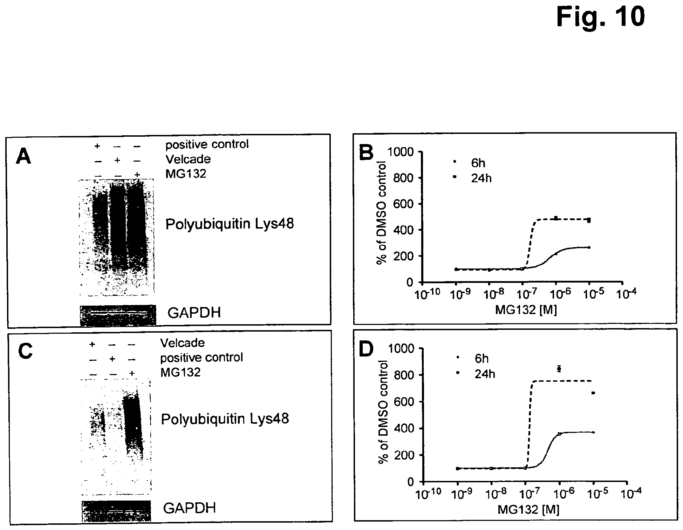

Experiments are performed according to the methods given above. The results of the experiments are shown in Figure 10:
Hek293 (A+B) or HepG2 (C+D) cells were cultured at 37°C, 5%CO₂ and treated with 1µM Velcade, MG132 (Proteasome inhibitors) or with DMSO as a vehicle control for 24h (**A+C**) or 6h and 24h (**B+C**) with MG132 in a concentration dependend manner. Cell lysates were analyzed with Western Blot analysis (A+C) or a Polyubiquitin (Lys48) specific Luminex® xMAP® assay (B+D) to determine Polyubiquitin (Lys48) levels. Data are shown as % of DMSO control.

### Protein Secretion, analyte: CHOP

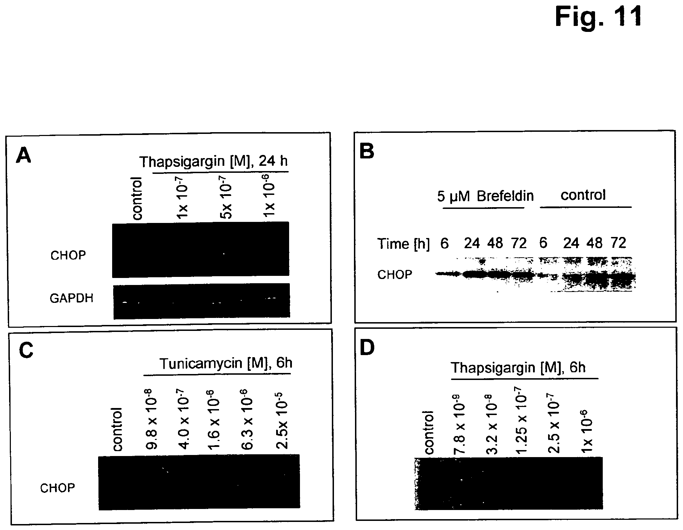

Rationale: CHOP is upregulated during ER stress. Increased CHOP expression indicates disturbance of the protein secretion process. Experiments are performed according to the methods given above. The results of the experiments are shown in Figure 11:
HepG2 (A, B) and HEK293 (C, D) cells were cultured at 37°C, 5%CO₂ and treated with indicated concentrations of Thapsigargin, Brefeldin A or Tunicamycin (respectively with EtOH, MetOH or DMSO as controls) for indicated times. Cell lysates were prepared and changes in CHOP expression levels were analysed by Western Blot technique using antibodies specifically detecting CHOP or GAPDH (depicted only in A) as internal control. For analysis fluorescence imaging (Odyssey, LI-COR) was used.

### Autophagy, analyte: LC3

Rationale: Accepted marker protein for autophagy. Tracking the conversion of LC3-I to LC3-II is indicative of autophagic activity.

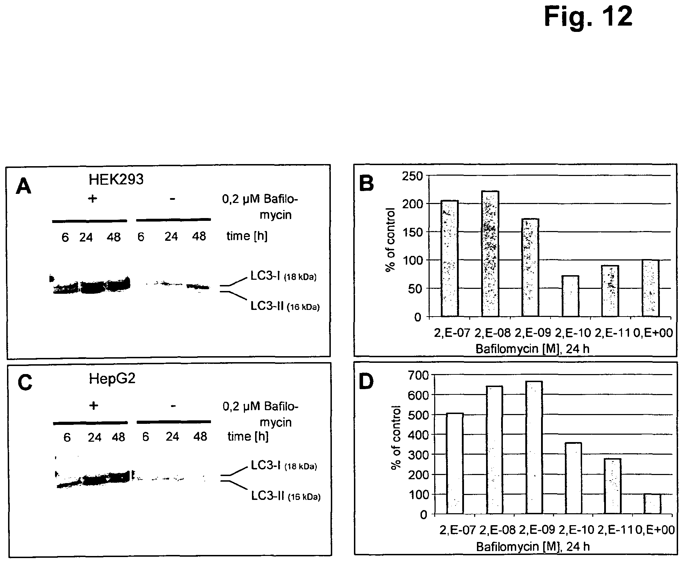

Experiments are performed according to the methods given above. The results of the experiments are shown in Figure 12:
HEK293 (A, B) or HepG2 (C, D) cells were cultured at 37°C, 5%CO₂ and treated with Bafilomycin or DMSO as vehicle control at the indicated concentrations and for the indicated time points. **A, C: Time-dependent change in LC3 levels, especially LC3-II.** Cell lysates were analyzed by western blot analysis using antibodies specifically detecting LC3-I and LC3-II (LC3-I: unlipidated form; LC3-II: lipidated form of LC3). **B, D: Dose-dependence of LC3 levels after treatment with different concentrations of Bafilomycin.** Bafilomycin was incubated for 24 hours with the cells at the indicated concentrations, cells were lysed and LC3 was detected by Western Blotting. After densitometric quantification of Western Blots the data were transformed to % of DMSO control.

### Metabolic Activity, analyte: Acetyl CoA carboxylase (pSer79)

Rationale: A drop in AMP/ATP ratio leads to activation of AMPK. Activation of AMPK leads to phosphorylation of ACC.

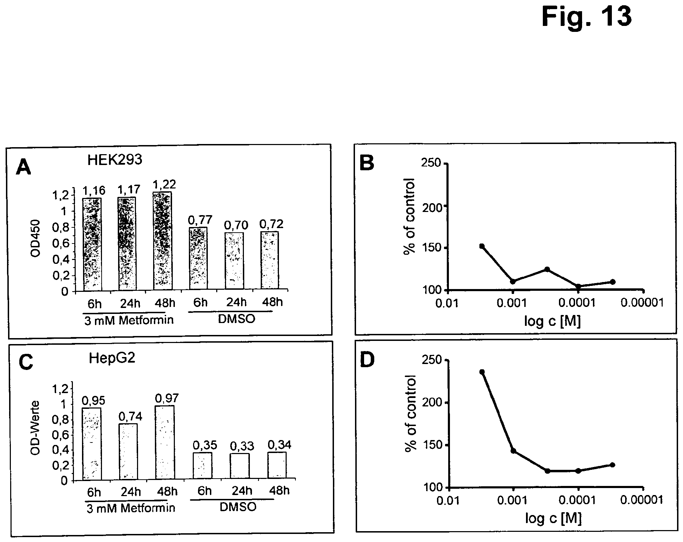

Experiments are performed according to the methods given above. The results of the experiments are shown in Figure 13:
HEK293 (A, B) or HepG2 (C, D) cells are cultured at 37°C, 5%CO₂ and treated with Metformin (a weak inhibitor of mitochondrial respiration) or DMSO as vehicle control at the indicated concentrations and for the indicated time points.

A, C: Time-dependent change in phospho-Acetyl CoA Carboxylase (p-Ser79) levels. Cell lysates were analyzed by ELISA (provider: Cell Signaling) using phospho-Acetyl CoA Carboxylase (p-Ser79) and Acetyl CoA Carboxylase-specific antibodies.

B, D: Dose-dependence of phospho-Acetyl CoA Carboxylase (p-Ser79) at different concentrations of Metformin. Metformin was incubated for 24 hours with the cells at the indicated concentrations, cells were lysed and phospho-Acetyl CoA Carboxylase (p-Ser79) was determined by using a specific ELISA assay (provider: Cell Signaling) and detecting the absorption at 405 nm.

### Exemplary multiplex measurement of 12 analytes with two panels

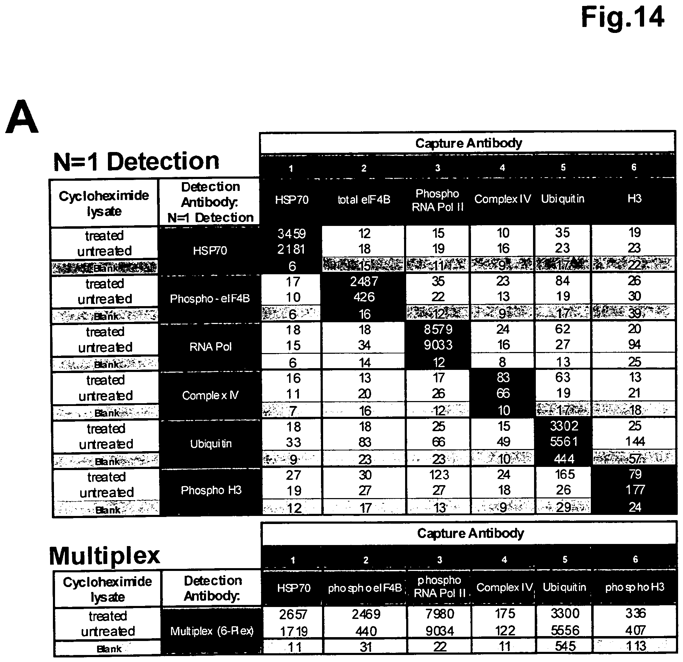

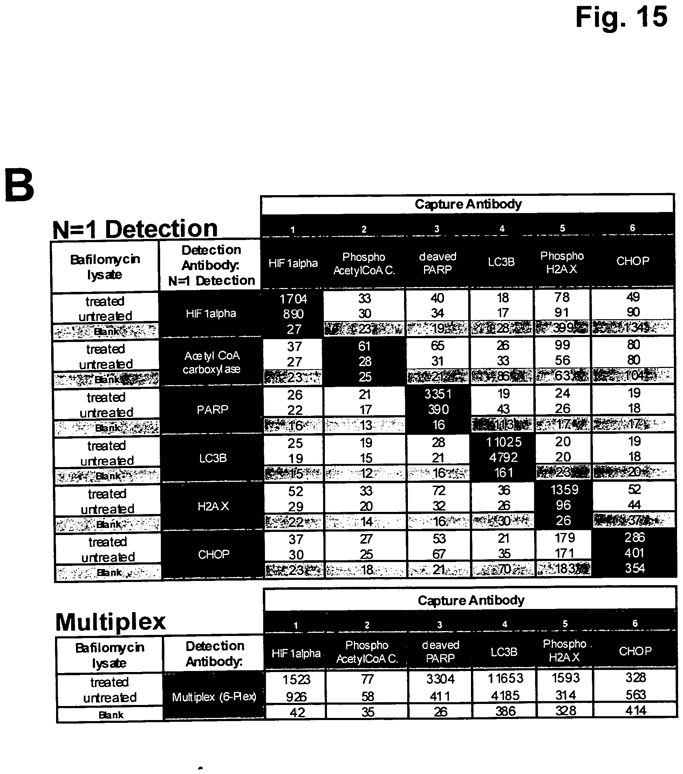

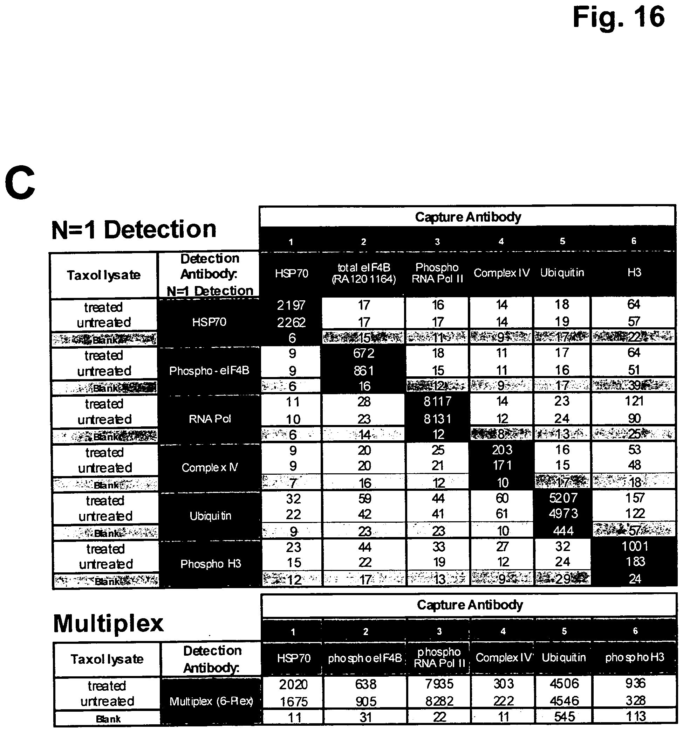

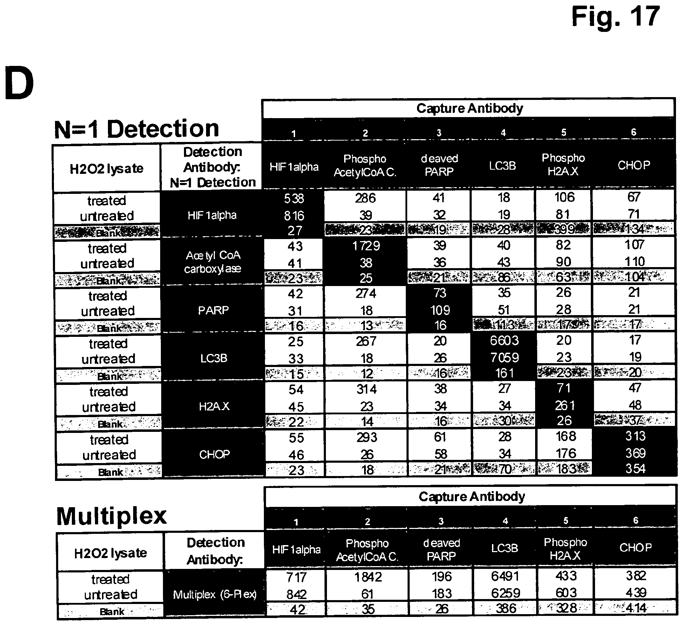

The results of the experiments are shown in Figures 14 to 17: Hep G2 cells are treated with different drugs: Figure 14 (**A**) Cycloheximide (1 µM, 24h) to induce elF4B phosphorylation, Figure 15 (**B**) Bafilomycin (0.2 µM, 48h) to induce LC3B conversion, Figure 16 **(C)** Taxol (0.1 µM, 24h) to induce histone H3 phosphorylation or Figure 17 **(D)** H₂O₂ (10 mM, 10 min) to induce Acetyl CoA Carboxylase phosphorylation. Cells are lysed with high concentration salt maltoside buffer (benzonase (0.25 units/µl)) and mean fluorescent intensities are measured for 12 analytes with two 6Plex panels **(A and B, C and D)** using a Luminex200 instrument. In panel one the following analytes are measured: Hsp70, elF4B (pSer422), RNA polymerase II (CTD pSer2), Complex IV, Ubiquitin (Lys48) and Histone H3 (pSer10). In panel two six additional analytes are measured: Hif1-alpha, Acetyl CoA carboxylase (pSer79), cleaved Parp, LC3B, Histone H2A.X (pSer139) and Chop. For each drug an "N=1 detection" and a "multiplex" experiment are performed. Samples of "N=1 detection" experiments include all capture beads (6 per panel), cell lysate, and one detection antibody (N=1). Samples of "multiplex" experiments include all capture beads (6 per panel), cell lysate, and all detection antibodies (6 per panel). MFI of the "N=1 detection" experiment and the "multiplex" experiment are compared to each other to analyse antibody cross-reactivity. Prior to that several antibody pairs for the detection of a single analyte are tested and antibodies with no or negligible cross-reactivity are picked for the final combination of antibodies in the multiplex panels. To give an example that drug-specific induction of an analyte can be measured with the multiplex panel: Analogue to figure 13 phosphorylation of Acetyl CoA Carboxylase can be induced by H₂O₂ treatment ("treated" 1842 MFI (multiplex), 1729 MFI (N=1 detection) and "untreated" 61 MFI (multiplex), 28 MFI (N=1 detection) (Figure 17, **D**). Treatment with Bafilomycin known to have insignificant effects on AcetylCoA Carboxylase does not induce phosphorylation of Acetyl CoA carboxylase (**C**).

### Abbreviations

elF4B = translation elongation initiation factor 4B
RNA Pol = DNA-dependent RNA polymerase II
H2A.X = histone H2A.X
Parp = Poly [ADP-ribose] polymerase 1
H3 = Histone H3
HSP70, HSP27 = heat shock protein 70, heat shock protein 27
Hif-1 alpha = hypoxia inducing factor 1 alpha
Chop = Gadd153 = growth arrest and DNA-damage-inducible protein GADD153
LC3 = microtubule-associated protein 1 light chain 3B
CHK1 = CHEK1= Serine/Threonine Protein Kinase (CHK1 = checkpoint homologue)
RB = Retinoblastom 1
TFIID, TFIIH, TFIIB = transcription factor II D, H oder B
CDC25 = serine/threonine protein phosphatase Cdc25
HMOX1 = heme oxygenase 1
PRDX2 = Peroxyredoxin 2
NBR1 = neighbor of BRCA1 gene 1
PDHE1 = E1 subunit of pyruvate dehydrogenase
TGS1 = trimethylguanosine synthase 1
RHOA = Ras homolog gene family, member A
DNM2 = dynamin 2
ARRB1 = Arrestin beta 1
SYNC1 = syncoilin 1
KRT15 = Keratin 15
ACTB = Actin beta
TIMP 1 = metallopeptidase inhibitor 1
FN1 = Fibronectin 1
ITGV = Integrin alpha V
TUBA1A = tubulin alpha 1.
KIF5B = kinesin family member 5B
SIRT2 = NAD-dependent deacetylase sirtuin-2
ELMO1 = Engulfment and cell motility protein 1
MYO1A = Myosin-Ia
AQP1 = aquaporin 1
CADM1 = cell adhesion molecule 1
STAG1 = Cohesin subunit SA-1
CNTNAP1 = contactin associated protein 1
EXOC4 = exocyst complex component 4
PDCD4 = Programmed cell death protein 4
YKT6 = Synaptobrevin homolog *YKT6*
CTD = carboxy terminal domain

## Claims

1. A method for determining the status of cells by
a) incubating the cells with the a potential drug
b) lysing the cells with a lysis buffer comprising dodecyl-maltoside
c) performing an assay for at least 4 parameters out of the following group of parameters
- DNA Integrity
- Cell Division
- Cell Death
- Transcriptional Activity
- Protein Biosynthesis
- Mitochondrial Activity
- Stress Response
- Oxidative Stress
- Protein Degradation
- Protein Secretion
- Autophagy
- Metabolic Activity
whereby the parameter DNA Integrity is measured by determining an analyte selected from the group of ATM, ATR, RAD-1, RAD-9, Hus-1, BRCA-1, BRCA-2, ATRIP, RPA-1, KU-70, KU-80, BARD-1, H2A.X (pSer139), CHK1 (pSer296), p53 (pSer15), CHK2 (pThr68), KAP1 (pSer824) and 53BP1 (pThr543),
the parameter Cell Division is measured by determining an analyte selected from the group of Cyclin-D, Cyclin-B, CDC-25A, CDC-25B, Aurora-A, Aurora-B, E2F-, Histone H3 (pSer10), Rb (pSer780, pSer795, pSer807/811) and Cdc2 (pTyr15),
the parameter Cell Death is measured by determining an analyte selected from the group of Puma, Bax, Bad, Bcl-2, Bcl-XL, Bcl-W, Apaf-1, Smac/Diablo, Noxa, AIF, Mcl-1, Cytochrom c, GADD34, BAG-1, Caspase-7, Caspase-8, Caspase-9, Caspase-10, Histone H2B (pSer14), Caspase-3, cleaved PARP and DFF-45,
the parameter Transcriptional Activity is measured by determining an analyte selected from the group of TFIID, TFIIH, TFIIB, SP1, PTEFb (positive transcription elongation factor b), RNA Pol II (pSer2/5), pan RNAPol II and Mcl-1,
the parameter Protein Biosynthesis is measured by determining an analyte selected from the group of PRKCA, MKNK1, MAPK1, mTOR, DIO2, Akt1, eIF4E, PPP1CA, MAPK3, eIF4G1, HSPB1, PTK2B, MKNK2, eIF2alpha (pSer51), elF4B (pSer422) and 4E-BP1 (pThr37/46),
the parameter Mitochondrial Activity is measured by determining an analyte selected from the group of enzymes of the citrate cyclus, Complex I, II, III, IV, V, NNT and frataxin,
the parameter Stress Response is measured by determining an analyte selected from the group of Hsp70, Hsp27, HSP60, HSP90 and Hsp27 (pS78/82),
the parameter Oxidative Stress is measured by determining an analyte selected from the group of PRDX1 (peroxiredoxin 1), GPX1, ANXA1, CCND1, DERL2, ERN1, ERO1L, GPX1, GSK3B, HDAC1, HERPUD1, HMOX1, HSPA5, LONP1, PPP1R15A, PRDX2, PXN, SPHK1, TXNRD1, WFS1, HIF-1 alpha and Ho-1,
the parameter Protein Degradation is measured by determining Polyubiquitin (Lys48),
the parameter Protein Secretion is measured by determining an analyte selected from the group of protein disulfide isomerase (PDI), Hsc70, calnexin, calreticulin, peptidyl-prolyl isomerase, N-acetylgalactosamine (GalNAc) transfer and CHOP,
the parameter Autophagy is measured by determining an analyte selected from the group of Beclin-1, VPS-34, PTEN, mTOR, PI-3 kinase, ULK-1, ULK-2, FIP-200, ATG-101, APG-3, APG-7, APG-12, LC3 and NBR-1,
the parameter Metabolic Activity is measured by determining an analyte selected from the group of AMPK (pThr172), Acetyl-CoA carboxylase (pSer79) and PDHE1 (pSer232, pSer239 pSer300)
and whereby a multiplexed assay format is used.

2. Method according to claim 1, **characterized in that** an assay is performed for at least 6 parameters out of the group of parameters.

3. Method according to one or more of claims 1 to 2, **characterized in that** in step b) the cells are lysed with a lysis buffer comprising a protease, a phosphatase inhibitor and a nuclease.

4. Method according to one or more of claims 1 to 3, **characterized in that** in step b) the cells are lysed with a lysis buffer comprising
- 1 to 100 mM dodecyl-maltoside
- 20 to 600 mM Magnesium chloride or a mixture of magnesium chloride with chlorides of one or more other alkali or alkaline earth metals
- at least 0.025 units of a nuclease
- a protease
- a phophatase inhibitor

5. Method according to one or more of claims 1 to 4 **characterized in that** in step c) a bead-based assay format is used.

6. Method according to one or more of claims 1 to 5 **characterized in that** in step c) the assay is performed with two or more different concentrations of the potential drug and/or for two or more different incubation times and/or with a combination of two or more potential drugs and/or with two or more different cell lines and/or in comparison to a vehicle control.

7. Method according to one or more of claims 1 to 6 **characterized in that** at least a second set of data for at least one compound with known effect is generated using the same assay and the results of both assays are compared.

8. Method according to one or more of claims 1 to 7 **characterized in that** in a step d) the set of data generated with the assay in step c) is compared with the sets of data contained in a data base.

9. Use of the method according to one or more of claims 1 to 8 to assess toxicity and/or side effects of potential drugs.

## Patentansprüche

1. Verfahren zur Bestimmung des Status von Zellen durch
a) Inkubieren der Zellen mit einem potenziellen Arzneistoff
b) Lysieren der Zellen mit einem Lysepuffer, der Dodecylmaltosid umfasst
c) Durchführen eines Assays hinsichtlich mindestens 4 Parametern aus der folgenden Gruppe von Parametern
- DNA-Integrität
- Zellteilung
- Zelltod
- Transkriptionsaktivität
- Proteinbiosynthese
- mitochondriale Aktivität
- Stressreaktion
- oxidativer Stress
- Proteinabbau
- Proteinsekretion
- Autophagie
- Stoffwechselaktivität,
wobei der Parameter DNA-Integrität durch Bestimmen eines Analyten, ausgewählt aus der Gruppe mit ATM, ATR, RAD-1, RAD-9, Hus-1, BRCA-1, BRCA-2, ATRIP, RPA-1, KU-70, KU-80, BARD-1, H2A.X (pSer139), CHK1 (pSer296), p53 (pSer15), CHK2 (pThr68), KAP1 (pSer824) und 53BP1 (pThr543), gemessen wird,
der Parameter Zellteilung durch Bestimmen eines Analyten, ausgewählt aus der Gruppe mit Cyclin-D, Cyclin-B, CDC-25A, CDC-25B, Aurora-A, Aurora-B, E2F-, Histon H3 (pSer10), Rb (pSer780, pSer795, pSer807/811) und Cdc2 (pTyr15), gemessen wird,
der Parameter Zelltod durch Bestimmen eines Analyten, ausgewählt aus der Gruppe mit Puma, Bax, Bad, Bcl-2, Bcl-XL, Bcl-W, Apaf-1, Smac/Diablo, Noxa, AIF, Mcl-1, Cytochrom c, GADD34, BAG-1, Caspase-7, Caspase-8, Caspase-9, Caspase-10, Histon H2B (pSer14), Caspase-3, gespaltenem PARP und DFF-45, gemessen wird,
der Parameter Transkriptionsaktivität durch Bestimmen eines Analyten, ausgewählt aus der Gruppe mit TFIID, TFIIH, TFIIB, SP1, PTEFb (positiver Transkriptionselongationsfaktor b), RNA Pol II (pSer2/5), pan RNAPol II und Mcl-1, gemessen wird,
der Parameter Proteinbiosynthese durch Bestimmen eines Analyten, ausgewählt aus der Gruppe mit PRKCA, MKNK1, MAPK1, mTOR, DIO2, Akt1, eIF4E, PPP1CA, MAPK3, eIF4G1, HSPB1, PTK2B, MKNK2, elF2alpha (pser51), elF4B (pSer422) und 4E-BP1 (pThr37/46), gemessen wird,
der Parameter mitochondriale Aktivität durch Bestimmen eines Analyten, ausgewählt aus der Gruppe der Enzyme des Citratzyklus, von Komplex I, II, III, IV, V, NNT und Frataxin, gemessen wird,
der Parameter Stressreaktion durch Bestimmen eines Analyten, ausgewählt aus der Gruppe mit Hsp70, Hsp27, HSP60, HSP90 und Hsp27 (pS78/82), gemessen wird,
der Parameter oxidativer Stress durch Bestimmen eines Analyten, ausgewählt aus der Gruppe mit PRDX1 (Peroxiredoxin 1), GPX1, ANXA1, CCND1, DERL2, ERN1, ERO1L, GPX1, GSK3B, HDAC1, HERPUD1, HMOX1, HSPA5, LONP1, PPP1R15A, PRDX2, PXN, SPHK1, TXNRD1, WFS1, HIF-1alpha und Ho-1, gemessen wird,
der Parameter Proteinabbau durch Bestimmen von Polyubiquitin (Lys48) gemessen wird,
der Parameter Proteinsekretion durch Bestimmen eines Analyten, ausgewählt aus der Gruppe mit Proteindisulfidisomerase (PDI), Hsc70, Calnexin. Calreticulin, Peptidyl-Prolyl-Isomerase, N-Acetylgalactosamin (GaINAc)-Transfer und CHOP, gemessen wird,
der Parameter Autophagie durch Bestimmen eines Analyten, ausgewählt aus der Gruppe mit Beclin-1, VPS-34, PTEN, mTOR, PI-3-Kinase, ULK-1, ULK-2, FIP-200, ATG-101, APG-3, APG-7, APG-12, LC3 und NBR-1, gemessen wird,
der Parameter Stoffwechselaktivität durch Bestimmen eines Analyten, ausgewählt aus der Gruppe mit AMPK (pThr172), Acetyl-CoA-Carboxylase (pSer79) und PDHE1 (pSer232, pSer239, pSer300), gemessen wird, wobei ein multiplexiertes Assayformat verwendet wird.

2. Verfahren nach Anspruch 1, **dadurch gekennzeichnet, dass** ein Assay für mindestens 6 Parameter aus der Gruppe von Parametern durchgeführt wird.

3. Verfahren nach einem oder mehreren der Ansprüche 1 bis 2, **dadurch gekennzeichnet, dass** in Schritt b) die Zellen mit einem Lysepuffer lysiert werden, der eine Protease, einen Phosphataseinhibitor und eine Nuklease umfasst.

4. Verfahren nach einem oder mehreren der Ansprüche 1 bis 3, **dadurch gekennzeichnet, dass** in Schritt b) die Zellen mit einem Lysepuffer lysiert werden, der Folgendes umfasst
- 1 bis 100 mM Dodecylmaltosid
- 20 bis 600 mM Magnesiumchlorid oder ein Gemisch von Magnesiumchlorid mit Chloriden eines oder mehrerer anderer Alkali- oder Erdalkalimetalle
- mindestens 0,025 Einheiten einer Nuklease
- eine Protease
- einen Phosphataseinhibitor.

5. Verfahren nach einem oder mehreren der Ansprüche 1 bis 4, **dadurch gekennzeichnet, dass** in Schritt c) ein Assayformat auf der Basis von Kügelchen verwendet wird.

6. Verfahren nach einem oder mehreren der Ansprüche 1 bis 5, **dadurch gekennzeichnet, dass** in Schritt c) der Assay mit zwei oder mehreren unterschiedlichen Konzentrationen des potenziellen Arzneistoffs und/oder während zweier oder mehrerer unterschiedlicher Inkubationszeiten und/oder mit einer Kombination von zwei oder mehr potenziellen Arzneistoffen und/oder mit zwei oder mehr verschiedenen Zelllinien und/oder im Vergleich zu einer Vehikelkontrolle durchgeführt wird.

7. Verfahren nach einem oder mehreren der Ansprüche 1 bis 6, **dadurch gekennzeichnet, dass** mindestens ein zweiter Satz von Daten für mindestens eine Verbindung mit bekannter Wirkung unter Verwendung desselben Assays erzeugt wird und die Ergebnisse beider Assays verglichen werden.

8. Verfahren nach einem oder mehreren der Ansprüche 1 bis 7, **dadurch gekennzeichnet, dass** in einem Schritt d) der mit dem Assay in Schritt c) erzeugte Satz von Daten mit den in einer Datenbank enthaltenen Sätzen von Daten verglichen wird.

9. Verfahren nach einem oder mehreren der Ansprüche 1 bis 8 zur Untersuchung der Toxizität und/oder der Nebenwirkungen potenzieller Arzneistoffe.

## Revendications

1. Méthode de détermination de l'état de cellules, par
a) l'incubation des cellules avec un médicament potentiel,
b) la lyse des cellules par un tampon de lyse comprenant du dodécyl-maltoside,
c) la mise en oeuvre d'un dosage pour au moins 4 paramètres choisis dans le groupe suivant constitué par les paramètres
- d'intégrité de l'ADN,
- de division cellulaire,
- de mort cellulaire,
- d'activité transcriptionnelle,
- de biosynthèse des protéines,
- d'activité mitochondriale,
- de réponse vis-à-vis d'un stress,
- de stress oxydatif,
- de dégradation des protéines,
- de sécrétion des protéines,
- d'autophagie,
- d'activité métabolique,
Où le paramètre d'intégrité de l'ADN est mesuré par la détermination d'un analyte choisi dans le groupe constitué par ATM, ATR, RAD-1, RAD-9, Hus-1, BRCA-1, BRCA-2, ATRIP, RPA-1, KU-70, KU-80, BARD-1, H2A.X (pSer139), CHK1 (pSer296), p53 (pSer15), CHK2 (pThr68), KAP1 (pSer824) et 53BP1 (pThr543),
le paramètre de division cellulaire est mesuré par la détermination d'un analyte choisi dans le groupe constitué par Cycline-D, Cycline-B, CDC-25A, CDC-25B, Aurora-A, Aurora-B, E2F-, Histone H3 (pSer10), Rb (pSer780, pSer795, pSer807/811) et Cdc2 (pTyr15),
le paramètre de mort cellulaire est mesuré par la détermination d'un analyte choisi dans le groupe constitué par Puma, Bax, Bad, Bcl-2, Bcl-XL, Bcl-W, Apaf-1, Smac/Diablo, Noxa, AIF, Mcl-1, Cytochrome c, GADD34, BAG-1, caspase-7, caspase-8, caspase-9, caspase-10, Histone H2B (pSer14), caspase-3, PARP clivé et DFF-45,
le paramètre d'activité transcriptionnelle est mesuré par la détermination d'un analyte choisi dans le groupe constitué par TFIID, TFIIH, TFIIB, SP1, PTEFb (facteur b positif de l'élongation de la transcription), ARN Pol II (pSer2/5), pan ARN Pol II et Mcl-1,
le paramètre de biosynthèse des protéines est mesuré par la détermination d'un analyte choisi dans le groupe constitué par PRKCA, MKNK1, MAPK1, mTOR, DIO2, Akt1, eIF4E, PPP1CA, MAPK3, eIF4G1, HSPB1, PTK2B, MKNK2, eIF2alpha (pSer51), elF4B (pSer422) et 4E-BP1 (pThr37/46),
le paramètre d'activité mitochondriale est mesuré par la détermination d'un analyte choisi dans le groupe constitué par les enzymes du cycle du citrate, le Complexe I, II, III, IV, V, NNT et la frataxine,
le paramètre de réponse vis-à-vis d'un stress est mesuré par la détermination d'un analyte choisi dans le groupe constitué par Hsp70, Hsp27, HSP60, HSP90 et Hsp27 (pS78/82),
le paramètre de stress oxydatif est mesuré par la détermination d'un analyte choisi dans le groupe constitué par PRDX1 (peroxyrédoxine 1), GPX1, ANXA1, CCND1, DERL2, ERN1, ERO1L, GPX1, GSK3B, HDAC1, HERPUD1, HMOX1, HSPA5, LONP1, PPP1R15A, PRDX2, PXN, SPHK1, TXNRD1, WFS1, HIF-1alpha et Ho-1,
le paramètre de dégradation des protéines est mesuré par la détermination de la Polyubiquitine (Lys48),
le paramètre de sécrétion des protéines est mesuré par la détermination d'un analyte choisi dans le groupe constitué par la protéine disulfure isomérase (PDI), Hsc70, la calnexine, la calréticuline, la peptidyl-prolyl isomérase, le transfert de N-acétylgalactosamine (GaINAc) et CHOP,
le paramètre d'autophagie est mesuré par la détermination d'un analyte choisi dans le groupe constitué par Becline-1, VPS-34, PTEN, mTOR, PI-3 kinase, ULK-1, ULK-2, FIP-200, ATG-101, APG-3, APG-7, APG-12, LC3 et NBR-1,
le paramètre d'activité métabolique est mesuré par la détermination d'un analyte choisi dans le groupe constitué par AMPK (pThr172), Acétyl-CoA carboxylase (pSer79) et PDHE1 (pSer232, pSer239, per300),
et où un format de dosage multiplex est utilisé.

2. Méthode selon la revendication 1, **caractérisée en ce qu'**un dosage est mis en oeuvre pour au moins 6 paramètres parmi le groupe de paramètres.

3. Méthode selon l'une ou plusieurs parmi les revendications 1 à 2, **caractérisée en ce que**, dans l'étape b), les cellules sont lysées par un tampon de lyse comprenant une protéase, un inhibiteur de phosphatase et une nucléase.

4. Méthode selon l'une ou plusieurs parmi les revendications 1 à 3, **caractérisée en ce que**, dans l'étape b), les cellules sont lysées par un tampon de lyse comprenant
- de 1 à 100 mM de dodécyl-maltoside,
- de 20 à 600 mM de chlorure de magnésium ou d'un mélange de chlorure de magnésium avec des chlorures d'un ou plusieurs autres métaux alcalins ou alcalino-terreux,
- au moins 0,025 unité d'une nucléase,
- une protéase,
- un inhibiteur de phosphatase.

5. Méthode selon l'une ou plusieurs parmi les revendications 1 à 4, **caractérisée en ce que**, dans l'étape b), on utilise un format de dosage à base de billes.

6. Méthode selon l'une ou plusieurs parmi les revendications 1 à 5, **caractérisée en ce que**, dans l'étape c), le dosage est mis en oeuvre avec deux, ou plus, concentrations différentes du médicament potentiel et/ou pour deux, ou plus, temps d'incubation différents et/ou avec une combinaison de deux, ou plus, médicaments potentiels et/ou avec deux, ou plus, lignées cellulaires différentes et/ou en comparaison avec un témoin de véhicule.

7. Méthode selon l'une ou plusieurs parmi les revendications 1 à 6, **caractérisée en ce qu'**au moins un deuxième set de données pour au moins un composé ayant un effet connu est généré en utilisant le même dosage, et les résultats des deux dosages sont comparés.

8. Méthode selon l'une ou plusieurs parmi les revendications 1 à 7, **caractérisée en ce que**, dans une étape d), le set de données généré avec le dosage de l'étape c) est comparé avec les sets de données contenus dans une banque de données.

9. Utilisation de la méthode selon l'une ou plusieurs parmi les revendications 1 à 8, destinée à évaluer la toxicité et/ou les effets secondaires de médicaments potentiels.
